(19) Europäisches Patentamt / European Patent Office / Office européen des brevets

(11) **EP 3 701 975 B1**

(12) **EUROPEAN PATENT SPECIFICATION**

(45) Date of publication and mention of the grant of the patent:
**27.12.2023 Bulletin 2023/52**

(21) Application number: **18869827.8**

(22) Date of filing: **25.10.2018**

(51) International Patent Classification (IPC):
***A61L 31/04*** (2006.01) ***A61K 9/14*** (2006.01)
***A61K 31/77*** (2006.01) ***A61K 47/46*** (2006.01)
***A61L 31/06*** (2006.01) ***A61L 31/14*** (2006.01)
***A61P 35/00*** (2006.01) ***C08L 1/02*** (2006.01)
***A61K 9/06*** (2006.01) ***A61K 47/36*** (2006.01)
***A61K 9/00*** (2006.01) ***A61K 9/16*** (2006.01)
***A61K 9/19*** (2006.01) ***A61K 47/32*** (2006.01)
***A61L 24/00*** (2006.01) ***A61L 24/08*** (2006.01)
***C08B 15/00*** (2006.01) ***C08B 15/10*** (2006.01)
***C08B 37/08*** (2006.01) ***C08L 5/08*** (2006.01)

(52) Cooperative Patent Classification (CPC):
(C-Sets available)
**C08B 15/10; A61K 9/0019; A61K 9/1075; A61K 9/1652; A61K 9/19; A61K 31/715; A61K 47/32; A61L 24/0031; A61L 24/08; A61P 35/00; C08B 15/005; C08B 37/003; C08L 1/02; C08L 5/08;** A61K 9/06; (Cont.)

(86) International application number:
**PCT/JP2018/039762**

(87) International publication number:
**WO 2019/082991 (02.05.2019 Gazette 2019/18)**

(54) **BIODEGRADABLE AND BIOMETABOLIC TUMOR SEALANT**

BIOLOGISCH ABBAUBARES UND BIOMETABOLISCHES TUMORDICHTMITTEL

AGENT DE SCELLEMENT DE TUMEUR BIODÉGRADABLE ET BIOMÉTABOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2017 JP 2017206227**

(43) Date of publication of application:
**02.09.2020 Bulletin 2020/36**

(73) Proprietor: **Tanaka, Takeo**
**Setagaya-ku**
**Tokyo 158-0083 (JP)**

(72) Inventors:
- **KONDOH, Shinae**
  **Tokyo 152-8550 (JP)**
- **TANAKA, Takeo**
  **Yokohama-shi**
  **Kanagawa 226-8510 (JP)**

(74) Representative: **Marks & Clerk LLP**
**15 Fetter Lane**
**London EC4A 1BW (GB)**

(56) References cited:
**WO-A1-2007/132852**
**WO-A1-2012/147255**
**WO-A1-2017/104840**
**JP-A- 2012 012 462**
**JP-A- 2015 512 950**
**US-A1- 2003 211 165**
**US-A1- 2007 237 830**
**US-B1- 8 377 484**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BAJPAI A K ET AL: "Investigation of magnetically enhanced swelling behaviour of superparamagnetic starch nanoparticles", BULLETIN OF MATERIALS SCIENCE, INDIAN ACADEMY OF SCIENCES, IN, vol. 36, no. 1, 3 April 2013 (2013-04-03), pages 15-24, XP035372155, ISSN: 0250-4707, DOI: 10.1007/S12034-013-0432-9 [retrieved on 2013-04-03]**
- **JUNG MIN PARK ET AL: "Design and characterisation of doxorubicin-releasing chitosan microspheres for anti-cancer chemoembolisation", JOURNAL OF MICROENCAPSULATION., vol. 29, no. 7, 14 May 2012 (2012-05-14), pages 695-705, XP055253488, GB ISSN: 0265-2048, DOI: 10.3109/02652048.2012.686526**
- **KISHIMURA Akihiro: "An emerging material 'PICsome': A hot zone between 'PEG' and 'PEG'", Drug Delivery System, vol. 1, no. 2 2016, pages 308-319, XP055698863, ISSN: 1881-2732 Retrieved from the Internet: URL:https://kyushu-u.pure.elsevier.com/en/publications/an-emerging-material-picsome-a-hot-zone-between-peg-and-peg**
- **"Start of preclinical test for nano device-Interruption of oxygen and nutrient flow into cancer cell", MEDIGEAR INTERNATIONAL CORPORATION, 8 February 2018 (2018-02-08), page 11, XP009520656,**


(52) Cooperative Patent Classification (CPC): (Cont.)
A61L 2400/12; A61L 2430/36

C-Sets
**A61L 24/08, C08L 1/26;**
**A61L 24/08, C08L 5/08**

## Description

Technical Field

The present invention relates to a particle device that cuts off and/or isolates tumor tissue from surrounding tissue including blood vessels by permeating through a blood vessel in a tumor site, selectively accumulating in and/or adhering to tumor tissue, swelling with water in the body, settling for a certain time to surround tumor and peritumoral cells and/or blood vessels, and covering a whole or a part of tumor, or a particle device that cuts off between blood vessels in a tumor site and tumor tissue.

Background Art

One of the minimally invasive treatments for liver cancer is hepatic artery embolization (TAE: trans-catheter arterial embolization). It is adopted in palliative care and terminal care, for example, in cases in which the liver is highly damaged and the disorder has progressed to inoperable. This is a therapy in which the feeding artery embolization is performed to stop the supply of nutrition and oxygen transported by blood and starve cancer to death. Hepatic cells are fed with nutrition and the like from the hepatic artery and portal veins, but malignantly transformed hepatic cells are fed with nutrition only from the hepatic artery. In contrast, it is said that normal cells do not die even after the hepatic artery embolization since the bloodstream from portal veins accounts for 70% in normal hepatic cells.

Moreover, in the hepatic arterial chemo-embolization (TACE: trans-catheter chemo-embolization), in which an anticancer agent is combined with the hepatic artery embolization, the embolus of the feeding artery with an embolic material is performed after the injection of a suspension of a contrast agent and an anticancer agent. At this time, the suspension may flow out into portal veins and there is a risk of causing embolus of the hepatic artery and portal veins to cause liver infarction. Furthermore, if the contrast agent, the anticancer agent, and the embolic material flow into the gallbladder or the pancreas, gallbladder inflammation or pancreatitis may be caused.

In view of such risk, embolic materials in which an anticancer agent is adsorbed onto spherical beads (microsphere) made of a polymer as a base material having a particle size of about tens micrometers to hundreds micrometers and having the function of slowly releasing the agent in the occluded blood vessel to suppress sudden release of the agent and the like into blood vessels, but not a suspension such as that described above, have also appeared.

There are two types of embolic materials: permanent embolic materials, which are made from a polymer as a raw material as described above and implanted permanently in the body, and transient embolic materials, which are made from a biodegradable material such as starch or gelatin as a raw material and degraded and metabolized. However, the embolus effect of many transient embolic materials is not sufficient and animal derived materials such as gelatin are regarded as contraindication in Japanese academic societies.

Meanwhile, the risk of permanent implanting is expected for permanent embolic materials, which has relatively large embolus effect. In the cases where the liver function is already damaged, such as hepatic cirrhosis, metabolic disorder occurs as the hepatic energy level decreases and liver failure becomes easy to be caused. To reduce the liver function damage as much as possible, it is required to restart the flow of blood through the hepatic artery and portal veins.

To solve the problem, Patent Literature 1 proposes an embolic material consisting of a biodegradable material that completely embolizes the blood vessel at the intended site without causing clogging of a catheter or an unintended blood vessel by aggregation, the bloodstream occlusion removed after a particular duration, and is degraded in the living body and metabolized or discharged out of the body.

In the embolotherapy (TAE/TACE), incomplete embolus situations caused by use of a transient embolic material having insufficient embolus effect or use of spherical embolus beads that generate gaps not only inhibit the bloodflow to normal cells to cause damages, but also lead the tumor environment to the hypoxia state to increase the risk to become a cause of malignant transformation.

According to the Non-Patent Literature 1, in the hypoxia state, even normal cells secrete proteins that suppress the function of immune cells against cancer and a part of immune cells act on the side of cancer. Non-Patent Literature 1 discloses that the hypoxia state causes a dreadful situation where, even if cancer cells of low malignancy are killed, cancer cells of high malignancy such as those that infiltrate tissue and extend transfer to another organ and increase their invasive potential.

In the cancer treatment, in general, it is extremely difficult to treat only cancer tissue. In surgical resection, surrounding normal tissue is also surgically removed to avoid leaving any cancer cells. In chemotherapies with anticancer agents, administration by infusion or intravenous injections causes heavy damage to normal tissue in the whole body. In irradiation, the damage to normal tissue locating in the path of radiation is unavoidable, although beams such as particle ray have been narrowed. This is why therapies with which only tumor tissue and tumor cells can selectively be treated are desired.

As described above, in therapies (TAE/TACE) using a microcatheter, the feeding artery of tumor can be embolized selectively among arteries around the tumor using a microcatheter, although much depends on the technique of the surgeon.

In chemotherapies in which a drug is administered, designs for selective delivery only to tumor have been increasingly attempted. One is to impart the molecular targeting function at a label specific for tumor to have an agent act on only tumor cells and referred to as the active targeting approach. There is also a passive targeting approach in which an agent is delivered according to the properties and structures characteristic of tumor tissue and tumor cells in contrast thereto.

A representative principle of the passive targeting is the Enhanced Permeability and Retention (EPR) effect.

Since endothelial cells of blood vessels of normal tissue are aligned without gaps, a macromolecular substance or the like does not leak halfway from blood vessels. However, tumor vessels are structurally incomplete and have large gaps between endothelial cells, which are said to be 50 nm to 500 nm. Particles having a particle size equal to or smaller than 500 nm can permeate through tumor vessels (Permeability).

Moreover, lymphatic vessels in tumor tissue are underdeveloped, and the circulation of substances is incomplete. Therefore, substances that have permeated through tumor vessels will consequently be retained and accumulated around the tumor (Retention).

Studies of Drug Delivery System (DDS) formulations employing the aforementioned EPR effect in drug delivery have become popular, but there are few examples put to practical use. For example, Leuplin and adriamycin are known. Even if a drug is delivered to near tumor, the effect as a drug is small, unless it is delivered into cells at a proper timing. Therefore, a further elaborate design for the permeation of the drug into cells is required.

There has been remarkable progress of medical materials in excellent biocompatibility, biodegradability, bioenvironmental responsiveness, and the like and artificial synthetic chemical substances that allow circumvention of risk of being derived from an animal have been developed.

Among amphiphilic compounds known to form liquid crystals as artificial synthetic chemical substances, low molecular weight liquid crystal compounds that form a self-organized non-lamella structure or the like that is superior in bioadhesiveness such as a cubic structure or a reversed hexagonal structure, but not a lamella structure, which is inferior in bioadhesiveness, such as a micellar structure, a vesicle structure, a dendrimer structure, or the like have been reported (Patent Literature 2, Patent Literature 3).

For example, Patent Literature 2 and 3 disclose development of a base material for injections (Patent Literature 2) and an adhesion preventing agent that makes use of bioadhesiveness (Patent Literature 3). However, there is no description about an embolic material or a sealant or use for embolus or sealing in these Patent Literature.

Moreover, Non-Patent Literature 2 and Patent Literature 4-5 disclose embolic materials for artery emboli using non-lamella liquid crystals. However, the embolus agents described in Non-Patent Literature 2 and Patent Literature 4 to 6 involves mixtures of amphiphilic compounds and water-soluble organic solvents as precursors of non-lamella liquid crystals and are emboli limited to use in blood vessels for controlling bloodflow or embolizing a blood vessel by forming bulk liquid crystal gel in blood vessels, but not intended for direct accumulation in or sealing of tumor tissue utilizing the EPR effect.

The present inventors have so far developed a biodegradable tumor sealant comprising a particle comprising a low molecular weight amphiphilic compound capable of forming non-lamella liquid crystals as a base material for the purpose of direct accumulation in or sealing of tumor tissue utilizing the EPR effect (Patent Literature 7). However, the biodegradable tumor sealant has not a little cytotoxicity and there has been a concern about damage to normal tissue.

Microspheres useful in the embolization treatment of tumors are described also in Patent Literature 8

Citation List

Patent Literature

Patent Literature 1:
Japanese Patent Laid Open No. 2004-313759
Patent Literature 2:
International Publication No. WO 2011/078383
Patent Literature 3:
International Publication No. WO 2014/178256
Patent Literature 4:
Chinese Patent No. 103040741
Patent Literature 5:
Chinese Patent No. 101822635

Patent Literature 6:
Chinese Patent No. 103536974
Patent Literature 7:
International Publication No. WO 2017/104840
Patent Literature 8:
US2007/0237830 A1

Non Patent Literature

Non Patent Literature 1:
Scientific American, Japan Edition, November, 2014, R. K. Jain, "An Indirect Way to Tame Cancer", p. 61
Non Patent Literature 2:
Science Portal China, No. 48, 'Research and development of new drugs - Yun Bin Wu, "Development of study on "cubic liquid crystal in controlled release preparation"' (in Japanese), September 6, 2010

Summary of Invention

Technical Problem

An object of the present invention is to provide a low invasive tumor sealant that is free of side effects of pharmaceutical preparations and radiation exposure risk, can be used in combination therapies with other therapies, or can be used to improve QOL of patients and that allows avoiding malignant transformation of tumor by a therapy to lead tumor cells to cell death by starving the cells, specifically, blocking oxygen and nutrition.

Solution to Problem

The present inventors have studied diligently to achieve the aforementioned object and have found, as a result, that particles made of superabsorbent polymer compounds as base materials permeate through gaps between endothelial cells of tumor vessels to accumulate in tumor tissue out of the blood vessels and swell by getting in contact with water in the body to form a gel composition and thereby block the supply of oxygen and nutrition to tumor and transmission of inducing factors from the tumor to lead the tumor cells to necrosis, thereby completing the present invention.
Accordingly, the present invention is as defined by the appended claims.

Advantageous Effects of Invention

According to the present invention, solid malignant tumor can be treated by a therapy involving starving tumor cells without any serious side effect of an anticancer agent or any major injury by surgery.

Brief Description of Drawings

[Figure 1] Figure 1 is a schematic diagram of the nano device (superabsorbent polymer compound) according to the present invention entering gaps between cells.
[Figure 2] Figure 2 illustrates the mechanism of action of the nano device.
[Figure 3] Figure 3 illustrates antitumor effect of the nano device on the basis of the difference between the amounts of luminescence due to the luciferin expression in the tumor to which the nano device according to the present invention is administered and in the tumor to which it is not administered in breast cancer cell-grafted mice.
[Figure 4] Figure 4 illustrates comparison between the change in tumor volume by the administration of the nano device according to the present invention to cancer-bearing mice of Figure 3 and the volume change of tumor without administration.
[Figure 5] Figure 5 illustrate the examination of antitumor effect in a cancer-bearing animal model. A polyacrylamide-based superabsorbent polymer compound (which may be also referred to as "MD2", herein) was administered as a nano device (sealant) to cancer tissue and the tumor size was measured over time.
[Figure 6] Figure 6 illustrate the examination of antitumor effect in a cancer-bearing animal model. A superabsorbent polymer compound (which may be also referred to as "MD3", herein) made from carboxymethylcellulose (CMC) as

a raw material was administered as a nano device (sealant) to cancer tissue and the tumor was extracted 8 days after the administration to measure the tumor size.

[Figure 7] Figure 7 illustrates the effect of blocking oxygen with a nano device (MD3).

[Figure 8] Figure 8 illustrates the effect of blocking protein/nutrient with a nano device (MD3) in vitro. The percentage of blocking transmission of protein with the nano device using FBS as a source of protein/nutrient is illustrated.

[Figure 9] Figure 9 illustrates the effect of blocking hemoglobin with a nano device (MD3) in vitro. The percentage of blocking transmission of hemoglobin with the nano device using FBS as a source of hemoglobin is illustrated.

[Figure 10] Figure 10 illustrates the effect of blocking glucose with a nano device (MD3) in vitro. The percentage of blocking transmission of glucose with the nano device using FBS as a source of hemoglobin is illustrated.

[Figure 11] Figure 11 illustrates the ischemia effect (decrease in oxygen concentration) in tumor vessels with a nano device (MD3) in breast cancer cell-grafted mice.

[Figure 12] Figure 12 illustrates a result of measurement of intratumoral bloodflow reduction by angiography using ICG.

## Description of Embodiments

The present invention will be described in detail below.

The tumor sealant according to the present invention is defined by the claims and is a particle made of a superabsorbent polymer compound as a base material and having a particle size that allows the superabsorbent polymer compound to permeate through gaps between endothelial cells of tumor vessels and the particle is characterized in that this particle permeates through the gaps, then attaches to tumor tissue in the vicinity of the gaps with its high bioadhesiveness, then swells by getting in contact with water in the body, and forms a barrier of a gel composition to block the supply of oxygen and nutrition to the tumor and the transmission of inducing factors from the tumor, and thereby leads the tumor cells to necrosis and the attached particle is subsequently degraded and/or metabolized in the living body. Moreover, while the tumor sealant according to the present invention has the aforementioned characteristics, it also has the effect of reducing the blood oxygen concentration and nutrition supply at the tumor site by pressing feeding vessels to the tumor to prevent the bloodflow and causing the necrosis of tumor cells, and therefore the tumor sealant according to the present invention also functions as a bloodflow preventing agent having the above-mentioned additional effect.

### 1. Superabsorbent polymer compound

The tumor sealant according to the present invention contains a superabsorbent polymer compound that swells with water and can form a gel composition and may be biodegradable and/or biometabolizable. As used herein, the term "superabsorbent" means a property of a material whose water absorption amount or swelling rate is at least 2 to 10000 times of the weight or volume of its dried base material. Moreover, the term "polymer" compounds usually means polymers composed of a repeat structure of a plurality of monomers, but herein refers to those including further cross-linked polymers. The molecular weight of the polymer compound is typically about 10 kDa to 2000 kDa, but not limited thereto, and may be changed as appropriate depending on the purpose of use. The superabsorbent polymer compound used in the present invention may be a compound represented by formula (1) described below as well as: polyacrylamide, glycosaminoglycan, cross-linked glycosaminoglycan, collagen, cross-linked collagen, polyacrylic acid, polymethylmethacrylate, polyvinyl alcohol, poly-L-lactic acid, or a derivative thereof. Moreover, the swelling ratio of the superabsorbent polymer compound used in the present invention is 2 to 10000 fold in weight ratio or volume ratio and may be, for example, 2 to 1000 fold, 2 to 500 fold, 2 to 300 fold, 2 to 200 fold or 2 to 100 fold (an upper limit of 90 fold, 80 fold, 70 fold, 60 fold, 50 fold, 40 fold, 30 fold, 20 fold, 15 fold, 10 fold, 9 fold, 8 fold, 7 fold, 6 fold, 5 fold, 4 fold, 3 fold). X (spacer) in the compound represented by formula (1) described below is a molecule derived from a cross-linker and the swelling ratio and the swelling speed had a tendency to increase when the cross-linker was reduced in the synthesis of the compound (data not shown).

The superabsorbent polymer compound used as the tumor sealant of the present invention is not limited as long as it has the aforementioned characteristics, and preferably has a structure typically represented by the following formula (1):

PS1-X-PS2 (1)

wherein PS1 and PS2 are polysaccharides and X is a spacer. More specifically, the above "PS1" and "PS2" may be the same or different and are independently selected from the group consisting of β glucan, chitin, chitosan, and derivatives thereof and mixtures thereof. Those skilled in the art would understand that the present invention encompasses, for example, a superabsorbent polymer compound in which "PS1" is β glycan and "PS2" is chitin or a superabsorbent polymer compound in which "PS1" is β glycan and "PS2" is chitosan.

Examples of the polysaccharides expressed as PS1 and PS2 include β glucan, chitin, chitosan, amylose, starch, glycogen and derivatives thereof and mixtures thereof. The β glucan is not limited, but includes cellulose and the derivative of β glucan typically includes carboxymethylcellulose and TEMP (2,2,6,6-tetramethylpiperidine-1-oxyl)-oxidized cellulose

The spacer ("X") in the structure represented by the above formula (1) is a molecule from a cross-linker and not limited, as long as it can link the polysaccharides ("PS1" and "PS2") by cross-linking reaction. Moreover, X is not limited to be used between molecules, but may be used for cross-linking within a molecule. More specifically, preferred examples of cross-linkers that can link polysaccharides by cross-linking reaction in the present invention are one or more selected from the group consisting of: carboxylic anhydrides, for example, 1,2,3,4-butanetetracarboxylic dianhydride (BTCA), polyethylene glycol diglycidyl ether (PEGDE), biphenyltetracarboxylic dianhydride (BPDA), diphenylsulfonetetracarboxylic dianhydride (DSDA), DSDA, and epichlorohydrin. For the superabsorbent polymer compound used in the present invention to be superabsorbent, it is preferred that the superabsorbent polymer compound has the same kind or different kinds of dissociative (ionic) functional groups in the molecule. A typical example of the dissociative functional group is -COO-Na+, and such bound and free anions are necessary for binding and maintaining water molecules. Moreover, those skilled in the art would be able to select the kind, the amount used, and the ratio of the polysaccharides, and the kind, the amount added, and the ratio of the cross-linker(s) and the density of crosslinking (may simply be referred to as degree of crosslinking), and the like, as appropriate, for the purpose of adjusting the water absorption amount and the swelling rate of the superabsorbent polymer compound produced to preferred values.

Examples of the superabsorbent polymer compound that can form a gel composition are one or more selected from, but not limited to, the group consisting of:

[Chemical Formula 2]

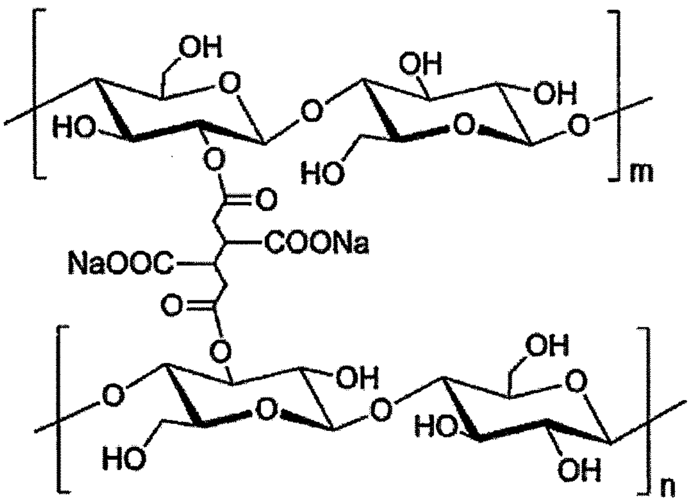

,

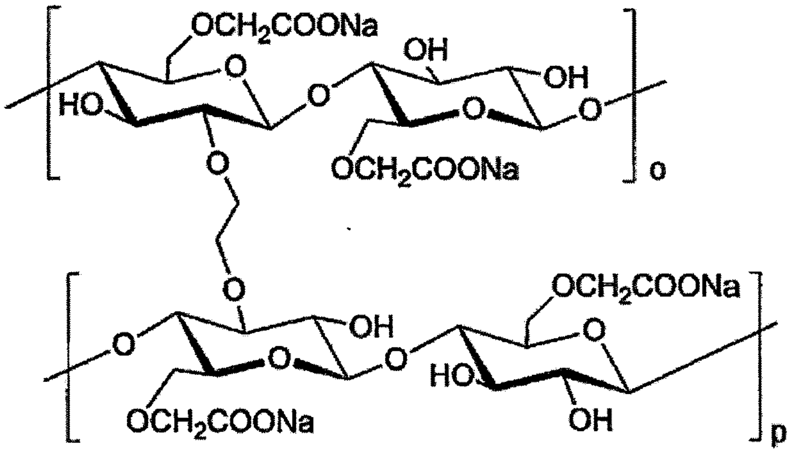

, and

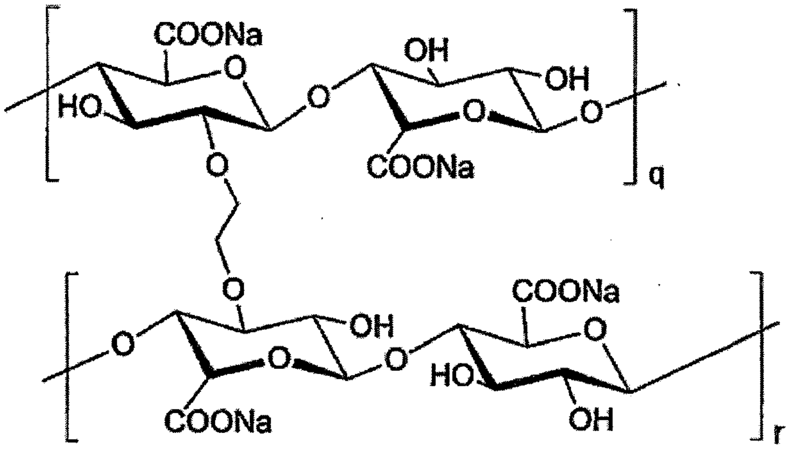

wherein, m to r are 100 to 200. For the synthesis of these superabsorbent polymer compounds, compounds may be produced using a well-known technique (see, for example, WO2012/147255, JP2012-12462A).

For example, a superabsorbent polymer compound made from cellulose as a raw material has a structure having chains cross-linked with tetracarboxylic acid. A method for producing the superabsorbent polymer compound is in brief as follows. The cellulose, a starting material, is dissolved in any of lithium chloride (LiCl)/N,N-dimethyl acetamide (DMAc), LiCl/N-methylpyrrolidone (NMP), tetrabutylammonium fluoride (TBAF)/dimethylsulfoxide (DMSO), and an ester-crosslinking reaction of the cellulose and a polycarboxylic anhydride is caused in the presence of N,N-dimethyl-4-aminopyridine (DMAP) as a catalyst at room temperature and atmospheric pressure. The polycarboxylic anhydride may preferably be 1,2,3,4-butanetetracarboxylic dianhydride (BTCA) or 3,3',4,4'-diphenylsulfonetetracarboxylic dianhydride (DSDA). By this ester-crosslinking reaction, the esterification between hydroxyl groups in the cellulose and the carboxylic

anhydride progresses to form ester-crosslinking between cellulose molecule chains and the carboxylic anhydride is simultaneously converted into the carboxyl group. Moreover, according to the present invention, the polymer chain is not limited to a naturally derived polymer chain, and derivatives thereof are also available. For example, as for cellulose, carboxymethylcellulose (CMC), sodium CMC, and ammonium CMC may be used. The superabsorbent polymer material made from CMC as a raw material may be produced in reference to Example 2 described below.

The reaction product obtained by the aforementioned ester-crosslinking reaction is precipitated in an organic solvent such as methanol or acetone, and neutralized to pH 7 with a basic aqueous solution. By this neutralizing reaction, the carboxyl group produced is converted into carboxylate. By these operations, a biodegradable superabsorbent polymer having carboxylate playing a water-absorbing role and ester-crosslinked three-dimensional crosslinked structure playing a water-holding role is obtained.

Since the water-absorbing and water-holding performances of cross-linked cellulose, cross-linked chitin, and the like depend on the cross-linking density (for example, polycarboxylic acid cross-linking density), superabsorbent polymer compounds having properties suitable for various application can be obtained by controlling the concentration of the prepared cross-linker, a reaction solvent, the degree of polymerization of cellulose or like that is a raw material, and the like.

As for the starting raw material, besides cellulose, a polysaccharide such as chitin, chitosan, amylose, or the like, or a mixture thereof may be used to obtain a superabsorbent polymer having similar performance. However, if chitosan or a polysaccharide is used as the starting raw material, a mixed system of an acid aqueous solution and an organic solvent, for example a mixed solvent of 10% acetic acid aqueous solution/methanol/NMP at 1:1:1, may be used as a solvent. Cellulose with a degree of polymerization of 1500 or higher such as cotton cellulose, among others, is preferable since biodegradable superabsorbent polymers having water-absorbing performance equal to or higher than currently commercially available products will be obtained by using such cellulose.

2. Properties of superabsorbent polymer compound

The superabsorbent polymer compound used in the biodegradable and/or biometabolizable tumor sealant according to the present invention does swell and form a gel composition upon getting in contact with water. The superabsorbent polymer compound exhibits excellent stability under a wide range of environmental conditions, as described above. In use of the aforementioned superabsorbent polymer compound as a tumor sealant, the phenomenon in which the superabsorbent polymer compound swells with water in the body to tens- to hundreds-fold volume of gel substance in comparison with the original volume, and thereby physically adheres to the tumor cell surface is used.

Examples of aqueous vehicles that can form gel formed by swelling of the superabsorbent polymer compound according to the present invention include, but is not particularly limited to, water such as sterile water, purified water, distilled water, ion exchanged water, ultra pure water; aqueous electrolyte solutions such as physiological saline, aqueous sodium chloride solutions, aqueous calcium chloride solutions, aqueous magnesium chloride solutions, aqueous sodium sulfate solutions, aqueous potassium sulfate solutions, aqueous sodium carbonate solutions, aqueous sodium acetate solutions; buffer solutions such as phosphate buffer solutions, tris hydrochloric acid buffer solutions; aqueous solutions containing water-soluble organic substances such as glycerin, ethylene glycol, ethanol; aqueous solutions containing sugar molecules such as glucose, sucrose, maltose; aqueous solutions containing water-soluble polymers such as polyethyleneglycol, polyvinyl alcohol; aqueous solutions containing surfactants such as octyl glucoside, dodecyl maltoside, Pluronic (polyethyleneglycol/polypropyleneglycol/polyethyleneglyco l copolymer); body fluids such as intracellular solutions, cell external solutions, stroma liquid, lymph, spinal fluid, blood, gastric juice, serum, plasma, saliva, tears, sperm, urine, and the like.

3. Biodegradable and/or biometabolizable tumor sealant

The biodegradable and/or biometabolizable tumor sealant according to the present invention is a nano particle made of a superabsorbent polymer compound as a base material and having a particle size that allows the nano particle to permeate through gaps between endothelial cells of tumor vessels, the nano particle is characterized in that this particle permeates through the gaps, then attaches to tumor tissue in the vicinity of the gaps with its high bioadhesiveness, then swells by getting in contact with water in the body, and forms a gel composition to block the supply of oxygen and nutrition to the tumor and the transmission of inducing factors from the tumor, and thereby leads the tumor cells to necrosis and the attached particle is subsequently degraded and/or metabolized in the living body.

Since the tumor sealant according to the present invention is required to have a size of the nano order that allows the tumor sealant to permeate through gaps between endothelial cells of tumor vessels, as described above, the particulation in the production of the nano particle is preferably performed during the process of ester-polymerization reaction of the superabsorbent polymer compound that is the base material. This nano particulation is not particularly limited, but may be performed using microfluidics. The microfluidics is highly preferable since the microfluidics makes

it possible to control the quantity of reaction by adjusting the flow rates of reaction substances and the flow velocity and therefore the particle size can be controlled by performing the particulation in accordance with the amount of polymer produced as the polymerization reaction progresses. Moreover, the use of the microfluidics has advantages including, in addition to the relatively easy control of particle size described above, advantages of the particle shape being near spherical and addition of a chemical modification step being easy. However, the nano particle prepared by microfluidics is in a state of hydrogel containing water and therefore preferably freeze-dried to make it solid fine powder.

Since the nano particle swells when it gets in touch with water, it is preferable that the nano particle is kept in an oily solution to block water and an emulsion of the lipid and an aqueous vehicle is prepared. The aqueous vehicle for preparing the nano particle in a form of emulsion is not particularly limited, and examples thereof include water such as sterile water, purified water, distilled water, ion exchanged water, ultra pure water; aqueous electrolyte solutions such as physiological saline, aqueous sodium chloride solutions, aqueous calcium chloride solutions, aqueous magnesium chloride solutions, aqueous sodium sulfate solutions, aqueous potassium sulfate solutions, aqueous sodium carbonate solutions, aqueous sodium acetate solutions; buffer solutions such as phosphate buffer solutions, tris hydrochloric acid buffer solutions; aqueous solutions containing water-soluble organic substances such as glycerin, ethylene glycol, ethanol; aqueous solutions containing sugar molecules such as glucose, sucrose, maltose; aqueous solutions containing water-soluble polymers such as polyethyleneglycol, polyvinyl alcohol; aqueous solutions containing surfactants such as octyl glucoside, dodecyl maltoside, Pluronic (polyethyleneglycol/polypropyleneglycol/polyethyleneglyco l copolymer); body fluids such as intracellular solutions, extracellular solutions, stroma liquid, lymph, spinal fluid, blood, gastric juice, serum, plasma, saliva, tears, sperm, urine, and the like.

The base material of the particle may only contain the aforementioned superabsorbent polymer compound, and may further include a functional substance (for example, a drug, a contrast agent, a dye, or the like). In practice, either or both of a contrast agent and a dye may be included to visualize sealing conditions of the tumor sealant (specifically, conditions of attaching to and implanted in a tumor site after the permeation through blood vessel and/or the process of gradually degrading in the living body after adhesion). Since the tumor sealant according to the present invention may contain a contrast agent and/or a dye, as seen above, the tumor sealant may be clinically applied to diagnosis of cancer.

Examples of a contrast agent for X-ray CT include Lipiodol, which is an iodinated hydrophobic contrast agent, Iopamiron, which is an aqueous contrast agent, and the like; examples of a contrast agent for MRI include Gd (gadolinium)-based or Fe (iron)-based magnetic materials; and examples of a contrast agent for echo include ultrasonic liposomal particles. These may include any one or mixture of two or more.

Moreover, examples of a dye for securing visibility before and during operation include fluorescent dyes such as coumalin, which is hydrophobic, fluoresceine, which is water-soluble, pyranine, cyanine; and photoluminescent dyes such as luciferin. These may include any one or mixture of two or more.

Furthermore, each one or more of the contrast agent and the dye may be included simultaneously.

The blending ratio of the aforementioned superabsorbent polymer compound and the contrast agent and/or the dye may be 100:1 to 1:100 and may be, for example, 95:5, 10:1, 90:10, 10:2, 80:20, 10:3, 70:30, 60:40, 50:50, 40:60, 30:70, 20:80, 10:90, 5:95. The blending ratio is preferably 100:1 to 50:50 and more preferably 10:1 to 10:3.

Usually in tumor tissue, tumor peripheral arteries include many neovessels and the vascular endothelial cells that constitute these blood vessels as well as cancer cells have lower adhesiveness with one another than normal cells and therefore there are several nanometers to several micrometers of gaps between the cells. The nano particles can be dispersed into the gaps. Figure 1 illustrates the comparison of the sizes of the tumor sealant according to the present invention, cells, and gaps in extracellular microenvironment in tumor tissue. The gaps are called stroma and composed of collagen, fibroblasts, blood vessels (neovessels in cancer tissue), lymphatic vessels, and the like. Lymphatic vessels are usually not developed in cancer tissue and the fluidity of particles is inhibited by the presence of collagen, fibroblasts, and the like in stroma of cancer microenvironment. The present invention makes use, as one of the mechanisms of action, of the enhanced permeability and retention (EPR) effect, in which particles permeate through these gaps between vascular endothelial cells and accumulate and settle in tumor tissue. Furthermore, the tumor sealant according to the present invention enters gaps between cancer cells, swells several hundred-fold with water in the body, presses neighboring cancer cells or blood vessels, and causes ischemia effect by covering the whole tumor (Figure 2).

The particles comprising a superabsorbent polymer compound as a base material are prepared using dry grinding techniques represented by jet mill; wet grinding techniques such as high pressure homogenizer, spraydrying, and freeze-drying; and emulsion polymerization techniques and microfluid techniques, as described below. The particle sizes are not even and various sizes are mixed, but the majority of particle sizes (which comprises 70% or more and more preferably 90% or more of the total particles and may optionally comprise 100%) are from 20 nm to 500 nm and particles of less than 20 nm or particles of more than 500 nm may also be present. Therefore, when particles are herein specified with particle sizes depending on the form of use, particles having a particle size of "20 nm to 500 nm" are not intended to exclude, for example, particles having a particle size of "10 nm to 200 nm", particles having a particle size of "300 nm to 700 nm", and the like. Meanwhile, examples of a particle size of "20 nm to 500 nm" are intended to include

"20 nm to 400 nm", "20 nm to 300 nm", "20 nm to 200 nm", "20 nm to 100 nm", "30 nm to 500 nm", "30 nm to 400 nm", "30 nm to 300 nm", "30 nm to 200 nm", "30 nm to 100 nm", "40 nm to 500 nm", "40 nm to 400 nm", "40 nm to 300 nm", "40 nm to 200 nm", "40 nm to 100 nm", "50 nm to 500 nm", "50 nm to 400 nm", "50 nm to 300 nm", "50 nm to 200 nm", "50 nm to 100 nm", "100 nm to 500 nm", "100 nm to 400 nm", "100 nm to 300 nm", "100 nm to 200 nm", and the like.

The aforementioned particle can typically be prepared by adding, in any order, one or more of the aforementioned superabsorbent polymer compound or a salt(s) thereof, a contrast agent and/or a dye (when included), a proper quantity of one or more of pharmaceutically acceptable surfactants, and a proper quantity of an aqueous vehicle (for example, physiological saline, water for injection), and the like, and stirring and homogenizing the mixture.

As for the pharmaceutically acceptable surfactants, any surfactants used in the field of pharmaceutical or cosmetics may be used and surfactants, such as, but not limited to, for example, Pluronic (for example, Pluronic F127, polyoxyethylene polyoxypropylene (200 EO) (70 PO)), polysorbate 80 (polyoxyethylene sorbitan oleate, Tween 80) may be used.

In one embodiment, the tumor sealant according to the present invention may comprise the aforementioned particle at a dispersable concentration (for example, 0.001 to 15% by weight) but it is determined by the subject to which it is administered and the dose.

As a method for confirming the particle, the structure of the particle can be analyzed, for example, by small angle X-ray scattering (SAXS) and cryo-TEM. Moreover, the particle size distribution of the particle is measured, by dynamic light scattering.

The particle according to the present invention can be prepared by mixing one or more superabsorbent polymer compounds with a surfactant or the like and stirring and homogenizing the mixture, as described above, but can also be prepared using ultrasonic homogenization and a microfluid technique, as indicated in Example 1 and 2, as described below. Here, the "microfluid technique" is a generic name of techniques handling fluid in microspace and devices used for the technique are called "microfluid devices". These are devices having flow pass structure typically having a depth and a width of around several micrometers to several hundred micrometers manufactured by applying semiconductor nanofabrication technique and precision machining technique. Herein, particles are produced using filtering by grinding or an emulsion polymerization technique and a microfluid technique, but particles are preferably produced using the technique since it makes it possible to stability prepare particles having a desired particle size. The particle size of particles can be measured using a common method well known to those skilled in the art.

4. Properties and characteristics of biodegradable tumor sealant

(1) Passability through syringe or microcatheter

The tumor sealant according to the present invention comprises an emulsion in which the aforementioned superabsorbent polymer compound is nano-particulated and dispersed in an oily vehicle, as described above. The passability through syringe or microcatheter is secured with this emulsion.

(2) Permeability through tumor vessels

Tumor neovessels specifically formed in tumor tissue have decreased pericytes (blood vessel pericytes), which cover the blood vessels, and therefore gaps between endothelial cells in the tumor vessels are enlarged in comparison with normal cells. There are particles that leak out through the gaps. The tumor sealant according to the present invention has sufficiently small particle size to pass the gaps. The size of gaps varies individually and depends on the type and the site of tumor, but is considered to be usually equal to or larger than 5 nm and equal to or smaller than 700 nm.

(3) Dispersibility of particles composed of those with different particle sizes

The gaps of tumor vessels vary in size from small to large. Since the tumor sealant according to the present invention is an emulsion having particle sizes that are not even, but have distribution with a range of several hundred nanometers, the tumor sealant can increase the accumulation density by permeating through any gaps.

(4) Tumor tissue accumulating property

Particles that have permeated through tumor vessels are released around tumor, as described above, but the lymphatic vessel network is underdeveloped in tumor tissue unlike normal tissue. Therefore, the particles are not transferred to other sites with a body fluid via a lymphatic vessel. Thus, the released particle, that is to say, the tumor sealant according to the present invention has an accumulating property to remain in the tumor site. The aforementioned (2) and (4) are collectively called the EPR effect.

(5) Blocking of oxygen and nutrition

Since the tumor sealant according to the present invention aggregates in the state of gel, tumor cells are cut off from nutrition and oxygen necessary for proliferation by sealing tumor tissue densely to lead to cell death (necrosis).

(6) Inhibition of transmission of transcription inducing factor in transduction system

From tumor tissue and cells, various inducing factors are released. For example, HIF (hypoxiainducible factor) release from cancer cells under the hypoxia stress is transmitted to VEGF (vascular endothelial growth factor) to promote tumor angiogenesis and increase the supply of nutrition and oxygen to tumor, contributing to proliferation. The tumor sealant according to the present invention can prevent the diffusion of transmitted factors released from these tumors by covering and confining tumor tissue without gaps and thereby prevent malignant transformation.

(7) Prompt metabolism and excretion

The tumor sealant according to the present invention is excreted out from the body via degradation or solubilization with enzymes in the body and further degradation in part after enough time/period of time to necrotize tumor cells.

(8) Visibility and visualizing property

Up to (5) are properties limited to basic functions of the tumor sealant according to the present invention, there are functions useful in the setting of use by surgeon added as functions in practical use.

The first one is visibility in viewing. The inclusion of a dye (including a fluorescent dye) reduces the risk of confusion with other pharmaceutical preparations and the surgical operation at a wrong position can be avoided by displaying as a marker the surgical site and range.

Second, the inclusion of a contrast agent makes it possible to confirm whether sufficient surgical operation is being provided or has been provided at correct location by monitoring with an imaging device during the operation. Furthermore, since the sealant settles in the tumor site without flowing out, dislike when only a contrast agent is used, the sealant has a visualizing property that makes it possible to monitor the state of tumor after the operation with an imaging device, without performing an operation to inject the contrast agent again.

(9) Embolization property

The tumor sealant according to the present invention can be used as an embolic material for artery emboli. As an embolic material, the risk of permanent implanting can be avoided as a transient embolic material exhibiting the embolization effect equivalent to a permanent embolic material and the synergistic effect of the tumor sealing effect and the embolization effect can be further expected.

5. Use of biodegradable tumor sealant

The tumor sealant prepared as described above can be used, for example, to diagnose and/or treat solid malignant tumor. As used herein, the "solid malignant tumor" includes squamous cell carcinoma, breast cancer, cutaneous lymphoma, hemangiosarcoma, hepatobiliary cancer, head and neck cancer, lung cancer, mesothelioma, mediastinal cancer, esophageal cancer, gastric cancer, pancreatic cancer, small intestinal cancer, colon cancer, colorectal cancer, large bowel cancer, anal cancer, renal cancer, urethral cancer, bladder cancer, prostate cancer, urethral cancer, penile cancer, testicular cancer, cancer of the gynecologic organ, ovarian cancer, cancer of the endocrine system, skin cancer, cancer of the central nervous system including the brain; soft tissue sarcoma and osteosarcoma; and skin and intraocular-derived melanoma. The form of use is not limited, but the tumor sealant may be used in a form of emulsion in which particles made of one or more superabsorbent polymers as a base material may be used in a form of emulsion dispersed in an oily vehicle or may be used as a pharmaceutical composition comprising the particle. Since the tumor sealant according to the present invention is not only applicable to diagnosis of cancer such as solid malignant tumor, but also applicable to treatment of cancer, as seen above, the tumor sealant can be referred to as a "theranostics device".

According to the present invention, the tumor sealant may be introduced to perform embolus of tumor and/or prevent bloodflow from the artery in the vicinity of the tumor using a syringe or a microcatheter. In one embodiment, the biodegradable tumor sealant according to the present invention can be administered, for example, locally in the vicinity of tumor using a syringe since the biodegradable tumor sealant can be used to treat needle-accessible cancer. Here, the dose, the concentration, the number and the frequency of dosing, and the like of the biodegradable tumor sealant can be determined as appropriate by surgeons and veterinarians in consideration of the sex, age, body weight of the

subject (test subject), the state of the affected part, and the like. Here, the subject treated by the biodegradable tumor sealant according to the present invention is preferably a mammal and examples of the mammal include, but not limited to, primates such as human and monkey, rodents such as mouse, rat, rabbit, guinea pig, cat, dog, sheep, pig, cow, horse, donkey, goat, ferret, and the like.

In Example 3 described below, 100 μL of a tumor sealant (Example 1) in an emulsion form is administered (specifically injected) to breast cancer cell-grafted mice from a blood vessel in the vicinity of tumor with a syringe, and the antitumor effect was obtained. As for human application, it corresponds to the administration of the biodegradable tumor sealant according to the present invention at an amount of around 10 ml to 200 ml per dose to, for example, human solid malignant tumor from a blood vessel in the vicinity of the tumor, when calculated simply from the body weight on the basis of these experimental results of the breast cancer cell-grafted mice. Moreover, the biodegradable tumor sealant according to the present invention may be administered several times (for example, 2 to 10 times) at intervals (for example, twice a day, once a day, twice a week, once a week, once in 2 weeks), as appropriate, until desired therapeutic effect is obtained. The dose (specifically injection volume) per dosing and the frequency of dosing of the biodegradable tumor sealant according to the present invention are not limited to those described above, but may be adjusted as appropriate and determined by a person skilled in the art (for example, a surgeon or a veterinarian).

When the tumor sealant according to the present invention is used as a pharmaceutical composition, the pharmaceutical composition may be used in a form in which a carrier, an excipient and/or a stabilizing agent, and the like, as appropriate are incorporated in addition to the tumor sealant as an active ingredient. Also disclosed is a kit comprising a tumor sealant, a solvent (physiological saline or the like), a carrier, and the like enclosed in a container (vial or the like) and an instruction, and the like is provided. Moreover, the tumor sealant according to the present invention encompasses a medical material. Examples

The present invention will be described specifically with reference to Examples, below. Meanwhile, the present invention is not limited to Example described below.

[Example 1] Production of superabsorbent polymer compound made from cellulose as raw material

1 g of cellulose (Sugino Machine Limited) was added to a solution of lithium chloride (LiCl)/N-methylpyrrolidone (NMP) (5 g LiCl/95g NMP) and dissolved by stirring the mixture sufficiently at room temperature for 2 days. 1.14 g of N,N-dimethyl-4-aminopyridine (DMAP) as a catalyst and 3.07 g of 1,2,3,4-butanetetracarboxylic dianhydride (BTCA) as a cross-linker were added and stirred at room temperature for 24 hours to react. By this ester-crosslinking reaction, cross-linking was formed between cellulose molecule chains. Subsequently, the reaction product was precipitated in 1 L of a methanol solution and then pH was adjusted to 7.0 using a 10% aqueous solution of sodium hydroxide. Subsequently, the precipitate was ground with a homogenizer and filtered with a glass filter. After the filtration, washing and filtration with methanol and water were repeated several times, and finally dried under conditions at 70 degrees Celsius and decreased pressure to obtain a cellulose superabsorbent polymer compound. Any of the reagents that were used was purchased from Tokyo Chemical Industry Co., Ltd, otherwise specified particularly (hereinafter, the same).

[Example 2] Synthesis of superabsorbent polymer compound made from carboxymethylcellulose (CMC) as raw material

While stirring 100 ml of a 1.5 M aqueous solution of sodium hydroxide in a beaker, 5 g of CMC (Sansho Co., Ltd.) was added little by little avoiding lumps to dissolve completely. 0.5 ml of polyethylene glycol diglycidyl ether (PEGDE) was added as a cross-linker and the mixture was stirred at room temperature for 10 minutes, and then incubated at 60°C for 3 hours for the cross-linking reaction. 200 ml of methanol was added to the reaction product hydrogel and wet-ground with a homogenizer. After grinding, granulated hydrogel particles were filtered with a glass filter. After the filtration, washing and filtration with methanol and water were repeated several times until pH of a washing solution becomes near neutral, and finally dried under conditions at 70 degrees Celsius and decreased pressure to obtain a CMC superabsorbent polymer compound.

[Example 3] Evaluation of bloodflow-preventing effect at tumor site

(1) Creation of breast cancer cell-grafted mice

4T1 breast cancer cells that stably express luciferase (4T1-Luc) were obtained and 0.04 ml of a $2.5\times10^7$ cells/ml PBS/Geltrex (R) = 1/1 (vol/vol) cell suspensions was subcutaneously grafted into Balb/c nu/nu nude mice (Charles River Laboratories Japan, Inc.).

(2) Bloodflow-preventing effect

The tumor sealant (emulsion) produced in Example 1 was locally administered at the murine tumor site where the above-mentioned breast cancer cells were grafted. After the administration, phosphate buffered saline containing D-luciferin, which is a substrate of luciferase, was administered by intraperitoneal administration to be circulated in bloodflow and the luciferase (Luc) activity of the aforementioned breast cancer cells was observed over time with an in vivo imaging device (IVIS) (Spectrum). At the tumor site, fluorescence is observed on the basis of the Luc activity due to the presence of blood luciferin. As shown in Figure 3A, while fluorescence due to the Luc expression in grafted breast cancer cells can be observed continuously in the non-emulsion-administration mice, the fluorescence decreased immediately after the administration in the emulsion-administration mice and it has therefore been suggested that the administration of the emulsion prevents the delivery of blood D-luciferin to tumor and the emulsion administration prevents the bloodflow to the tumor site. Moreover, the ratio of the Luc activity in the administration mice to that in the non-administration mice was calculated for this observation result and the change over time is shown in Figure 3B.

(3) Tumor suppression

The change of tumor volume when the emulsion was administered several times was examined. The change was observed over time using nude mice in which 4T1-Luc cells were grafted, as described in the above (1). The administration of the emulsion was conducted on the first time (Day 1), Day 2, Day 3, and Day 4. The change over time in tumor volume is illustrated in Figure 4. As apparent from Figure 4, the tumor volume of the emulsion administration mice decreased in comparison with the control given physiological saline. As to this, the decrease in tumor volume became prominent after about 4 times of administration.

[Example 4] Confirmation of antitumor effect in cancer-bearing animal model

In this Example, using mice in which breast cancer cells employed in Example 3 were subcutaneously grafted as a cancer-bearing animal model, and using a commercially available polyacrylamide-based superabsorbent polymer compound (particle size: 500 to 600 nm) (Sanyo Chemical Industries, Ltd.) (which may, hereinafter, be referred to as the "MD2") as a sealant, and a superabsorbent polymer compound (particle size: 600 to 1500 nm) (which may, hereinafter, be referred to as the "MD3") made from carboxymethylcellulose (CMC) as a raw material, the antitumor effect based on the barrier formation by these was examined.

(1) Antitumor effect

The tumor sealant (contained in physiological saline) containing the superabsorbent polymer compound and only physiological saline (control) were locally administered to the mice and the change in tumor growth was observed.

(a) Antitumor effect of polyacrylamide-based superabsorbent polymer compound (MD2)

As illustrated in Figure 5, tumor growth was observed over time in the control given only physiological saline (100 μl). In contrast with this, the administration of a solution in which a superabsorbent polymer compound was included in physiological saline (10 mg/ml) was possible to markedly suppress tumor growth. A significant difference between the both was found in the comparison on Day 6.

(b) Case of CMC-based superabsorbent polymer compound (MD3)

The results obtained by measuring the change in tumor volume between before and after the administration of the superabsorbent polymer are summarized in Figure 6. Figure 6A illustrates the result when only physiological saline was used as vehicle. On the basis of the tumor volume just before the administration, a tumor is extracted 8 days after administration and the tumor volumes were compared with each other. In the control receiving only the physiological saline, the tumor increased to 342%. In the subject receiving the superabsorbent polymer, the tumor volumes decreased to 69%. Furthermore, a result when physiological saline + Lipiodol was used as vehicle is illustrated in Figure 6B. Like a result of Figure 6A, the decrease in tumor volume was able to be observed, but the tumor suppressive effect was enhanced by inclusion of the Lipiodol.

(2) Sealing effect (oxygen blocking effect)

The effect of blocking oxygen supply to tumor tissue with a superabsorbent polymer compound (MD3) disclosed

herein was examined. To visualize the hypoxia state due to the superabsorbent polymer compound at tumor sites in laboratory animals, in vivo photoacoustic three-dimensional tomography MSOT (MultiSpectral Optoacoustic Tomography) (model name: MSOT inVision 256-TG, Summit Pharmaceuticals International Corporation) was used. This system visualizes the oxygen saturation of hemoglobin and oxyhemoglobin, which is abundant with oxygen, and deoxygenated reduced hemoglobin may thereby be distinguished and visualized in red and blue, respectively. The image in which the hypoxia state is visualized by the system is illustrated in Figure 7. In comparison of right and left tumors, the control tumor (left side) to which no superabsorbent polymer compound was administered was confirmed to be in a state in which oxygen was abundant (red). In contrast, the tumor (right side) to which the superabsorbent polymer compound was administered was visually recognized as a deoxygenated state (blue) and it was confirmed that the tumor site was in a hypoxia state. This indicated that the superabsorbent polymer compound disclosed herein has antitumor effect as a sealant.

[Example 5] Examination of the protein of the superabsorbent polymer compound in the in vitro and the ability to block the nutrient

The blocking of supply of various blood substances to tumor tissue by the superabsorbent polymer compound disclosed herein is considered to be caused by swelling of the superabsorbent polymer compound due to contact with water in the body. To examine this, in this Example, a superabsorbent polymer compound was gelated in vitro and the blocking effect was examined by using the passage of protein and nutrient through the gel (permeability) as an indicator. First, gel of a superabsorbent polymer compound (MD3) was prepared using PBS (-) (final concentration: 0.01 g/mL). Next, predetermined volumes (0.2, 0.5, and 1.0 ml) of the gel prepared as described above were injected into a 2.5 ml syringe with a cotton stopper at bottom. Subsequently, fetal bovine serum (FBS) (100% or 10%) was charged into the syringe on the gel. Subsequently, the FBS that passed gel is collected and the sealing effect of the gel was examined by comparing the amounts of components (protein/nutrient, hemoglobin, and hemoglobin) remained in the FBS with respective amounts of components in the control. The control was the FBS that passed a syringe with a cotton stopper at bottom without charging the gel.

(1) Blocking of protein/nutrient

The amount of protein remaining in the FBS that passed the gel and was recovered was measured with an ultraviolet spectrophotometer and determined based on a standard curve. The ratio of the protein blocked by the gel was calculated by comparing the amount of the protein present in the control FBS and the amount remaining in the FBS after passing the gel. More specifically, the ratio was determined by the following formulas:

$$\text{Protein blocked (\%)} = (\text{Amount of protein in control} - \text{Amount of protein remaining after passing gel}) \times 100/\text{Amount of protein in control}$$

The result is shown in Figure 8. When diluted FBS (10%) was used, the protein-blocking effect was seen markedly, since the gel did not reach saturation. When non-diluted FBS (100%) was used, the protein-blocking effect was small in comparison with the diluted FBS since the gel was in saturation. When MD3 with volumes of 0.5 ml and 1.0 ml was used, significant difference was found.

(2) Blocking of hemoglobin

In the same manner as the protein/nutrient-blocking experiment of the above (1), hemoglobin-blocking effect of gel was examined. The result was shown in Figure 9. When MD3 with volumes of 0.5 ml and 1.0 ml was used, significant difference was found in the hemoglobin-blocking effect.

(3) Blocking of glucose

In the same manner as the protein/nutrient-blocking experiment of the above (1), glucose-blocking effect of the gel was examined. The result is shown in Figure 10. When 1.0 mL of MD3 was used, about 80% of glucose was entrapped in the gel, but the effect varied markedly depending on the amount of gel used. Moreover, there was no difference in the blocking effect due to the difference in charged FBS concentration,

As such, it was found that the superabsorbent polymer compound disclosed herein can prevent the permeation of serum components such as protein/nutrient, glucose, and hemoglobin.

[Example 6] Measurement of oxygen concentration in tumor vessels and examination of ischemia effect

Using mice in which 4T1-Luc cells were grafted, the ischemia effect (reduction of oxygen concentration) of the administration of the superabsorbent polymer compound on tumor vessels was further examined. The measurement of the oxygen state in tumor by photoacoustic three-dimensional tomography one day after the administration of the superabsorbent polymer compound (MD3) indicated increase of reduced hemoglobin (blue) in the tumor tissue on the side (left) to which MD3 was administered (Figure 11). By this, it was observed that blood oxygen concentration was decreased in the tumor tissue and blocking of bloodflow was assumed.

Furthermore, using, as a contrast agent, indocyanine green (ICG) that emits photoluminescence with a peak wavelength of 845 nm when bound to a serum protein, the change in bloodflow was measured by measuring the bloodflow in tumor tissue over time with the photoluminescent signal from the contrast agent. One day after the administration, ICG (Dojindo Laboratories, 2.5 mg/mL, 100 μL (320 nmol)) was administered to the aforementioned mice given MD3, fluorescence was excited by LED (light emitting diode) irradiation, images were obtained using a near infrared light imaging device (PerkinElmer, IVIS Spectrum), and the photoluminescence signal was measured from the images (Figure 12). As a result, photoluminescence from ICG was observed in the tumor tissue to which no MD3 was administered and it was observed that there was bloodflow. In contrast, photoluminescence from ICG was not observed in the tumor tissue to which MD3 was administered and no bloodflow was observed (Figure 12A). The result of plotting of averaged ICG signals obtained from the images and digitized over time is shown in Figure 12B. While the signal was increased from immediately after the administration of ICG in the tumor tissue to which no MD3 was administered, the ICG signal was not increased in the tumor tissue to which MD3 was administered. From the foregoing results, it was found that the superabsorbent polymer compound can block bloodflow to tumor tissue. Industrial Availability

By using particles of the tumor sealant according to the present invention, it is expected to increase new choices of low invasive therapies for preventing metastasis or infiltration of cancer. Moreover, the particles have advantages in degradability and visualization function as embolus agents and the possibilities as new embolization agents for embolotherapies will be opened up. Furthermore, since the particles can include a contrast agent and/or a dye, the application to marking materials in cancer diagnosis and surgical operations can be broaden.

Specific embodiments of the present invention have been described herein for the purpose of illustration, but a person skilled in the art will readily understood that various modifications may be made.

## Claims

1. A tumor sealant comprising a particle comprising a superabsorbent polymer compound as a base material, for use in a method of treating a solid malignant tumor in a subject having the solid malignant tumor,

   wherein 70% or more of the total particles have a particle size of 20 nm to 500nm, as measured by a dynamic light scattering method;
   wherein the particle can swell by getting in contact with water in a body of the subject and the swelling ratio of the particle is at least 2 to 1,000 times of the weight or volume of its dried base material;
   wherein, in the method, the tumor sealant is introduced, using a syringe or a microcatheter, to perform embolus of tumor and/or prevent blood-flow from the artery in the vicinity of the tumor; and
   wherein, in the method, the particle permeates through gaps between endothelial cells of tumor vessels to accumulate in tumor tissue out of the blood vessels and then swells by getting in contact with water in the body of the subject to form a gel composition, thereby blocking the supply of oxygen and nutrition to tumor and transmission of inducing factors from the tumor to lead the tumor cells to necrosis.

2. The tumor sealant for use according to claim 1, wherein the superabsorbent polymer compound has a structure represented by the following:

   PS1-X-PS2 (1)

   wherein PS1 and PS2 are polysaccharides and X is a spacer.

3. The tumor sealant for use according to claim 2, wherein PS1 and PS2 may be the same or different and are independently selected from the group consisting of β glucan, chitin, chitosan, and derivatives thereof and mixtures

thereof.

4. The tumor sealant for use according to claim 3, wherein the β glucan or derivative thereof is cellulose or carboxymethylcellulose.

5. The tumor sealant for use according to any one of claims 2 to 4, wherein X is one or more polycarboxylic acid anhydrides.

6. The tumor sealant for use according to any one of claims 2 to 4, wherein X is derived from one or more cross-linkers selected from the group consisting of 1,2,3,4-butanetetracarboxylic dianhydride (BTCA), polyethylene glycol diglycidyl ether (PEGDE), biphenyltetracarboxylic dianhydride (BPDA), diphenylsulfonetetracarboxylic dianhydride (DSDA), PEG (polyethyne glycol) 400, PEG 2000, and epichlorohydrin.

7. The tumor sealant for use according to claim 1, wherein the superabsorbent polymer compound is one or more selected from the group consisting of:

[Chemical Formula 1]

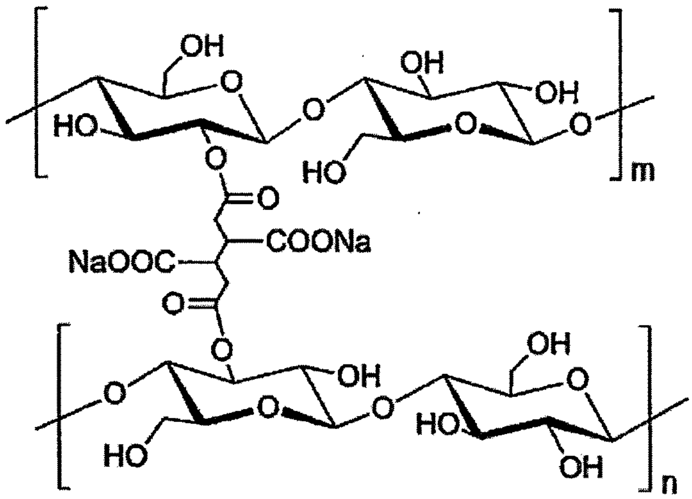

,

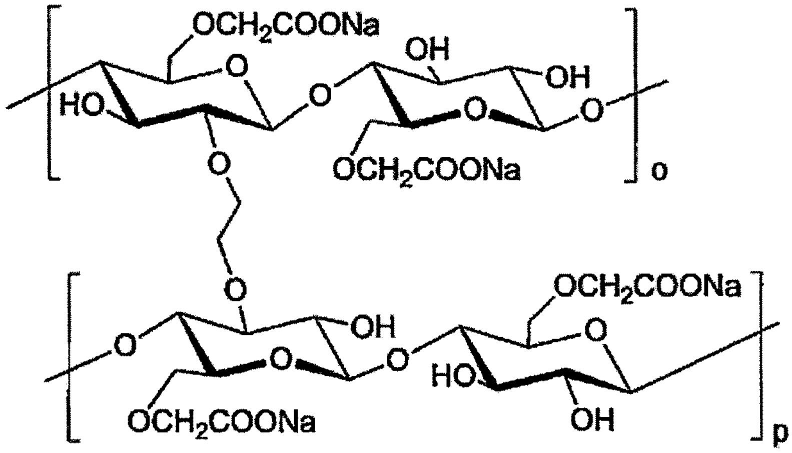

, and

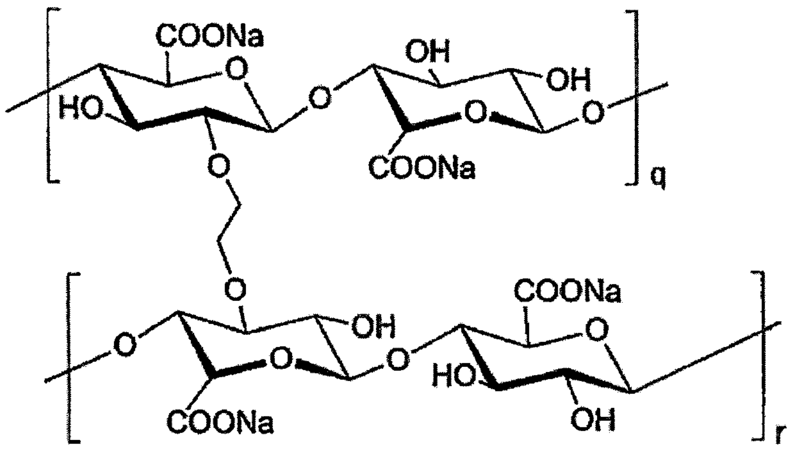

wherein m to r are 100 to 200.

**8.** The tumor sealant for use according to any one of claims 1 to 7, wherein 90% or more of the particle has a particle size of 20 nm to 500 nm.

**9.** The tumor sealant for use according to any one of claims 1 to 8, wherein the particle is in a form of emulsion.

**10.** The tumor sealant for use according to any one of claims 1 to 9, wherein the particle comprises a contrast agent and/or a dye.

**11.** The tumor sealant for use according to any one of claims 1 to 10, wherein the tumor sealant is in form of freeze-dried solid fine particles.

**12.** The tumor sealant for use according to any one of claims 1 to 10, wherein the tumor sealant is in an oily solution.

## Patentansprüche

**1.** Tumorversiegelungsmittel, umfassend ein Partikel, umfassend eine superabsorbierende Polymerverbindung als Basismaterial, zur Verwendung in einem Verfahren zur Behandlung eines soliden bösartigen Tumors bei einem Subjekt mit dem soliden bösartigen Tumor,

wobei 70 % oder mehr der gesamten Partikel eine Partikelgröße von 20 nm bis 500 nm aufweisen, wie mit einem dynamischen Lichtstreuungsverfahren gemessen;
wobei das Partikel anschwellen kann, indem es mit Wasser in einem Körper des Subjekts in Kontakt kommt und das Schwellverhältnis des Partikels mindestens das 2- bis 1 000-fache des Gewichts oder Volumens seines getrockneten Basismaterials beträgt;
wobei bei dem Verfahren das Tumorversiegelungsmittel unter Verwendung einer Spritze oder eines Mikrokatheters eingeführt wird, um eine Embolie des Tumors durchzuführen und/oder einen Blutfluss aus der Arterie in der Nähe des Tumors zu verhindern; und
wobei bei dem Verfahren das Partikel durch Lücken zwischen Endothelzellen von Tumorgefäßen hindurchdringt, um sich im Tumorgewebe außerhalb der Blutgefäße anzusammeln, und dann anschwillt, indem es im Körper des Subjekts mit Wasser in Kontakt kommt, um eine Gelzusammensetzung zu bilden, wodurch die Zufuhr von Sauerstoff und Nahrung zum Tumor und die Übertragung von Induktionsfaktoren vom Tumor blockiert werden, um die Tumorzellen zur Nekrose zu führen.

**2.** Tumorversiegelungsmittel zur Verwendung nach Anspruch 1, wobei die superabsorbierende Polymerverbindung eine Struktur aufweist, die dargestellt wird durch Folgendes:

PS1-X-PS2 (1)

wobei PS1 und PS2 Polysaccharide sind und X ein Spacer ist.

**3.** Tumorversiegelungsmittel zur Verwendung nach Anspruch 2, wobei PS1 und PS2 gleich oder verschieden sein können und unabhängig ausgewählt sind aus der Gruppe bestehend aus β-Glucan, Chitin, Chitosan und Derivaten davon und Mischungen davon.

**4.** Tumorversiegelungsmittel zur Verwendung nach Anspruch 3, wobei das β-Glucan oder das Derivat davon Cellulose oder Carboxymethylcellulose ist.

**5.** Tumorversiegelungsmittel zur Verwendung nach einem der Ansprüche 2 bis 4, wobei X ein oder mehrere Polycarbonsäureanhydride ist.

**6.** Tumorversiegelungsmittel zur Verwendung nach einem der Ansprüche 2 bis 4, wobei X von einem oder mehreren Vernetzern abgeleitet ist, die aus der Gruppe ausgewählt sind, die aus 1,2,3,4-Butantetracarbonsäuredianhydrid (BTCA), Polyethylenglykoldiglycidylether (PEGDE), Biphenyltetracarbonsäuredianhydrid (BPDA), Diphenylsulfontetracarbonsäuredianhydrid (DSDA), PEG (Polyethynglykol) 400, PEG 2000 und Epichlorhydrin besteht.

**7.** Tumorversiegelungsmittel zur Verwendung nach Anspruch 1, wobei die superabsorbierende Polymerverbindung eine oder mehrere ist, ausgewählt aus der Gruppe bestehend aus Folgendem:

[Chemische Formel 1]

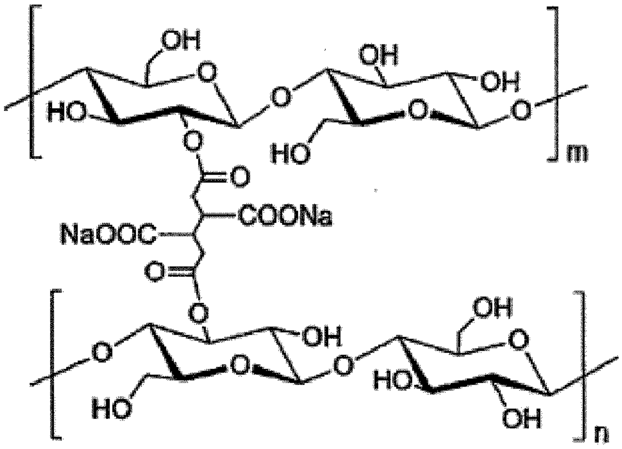

,

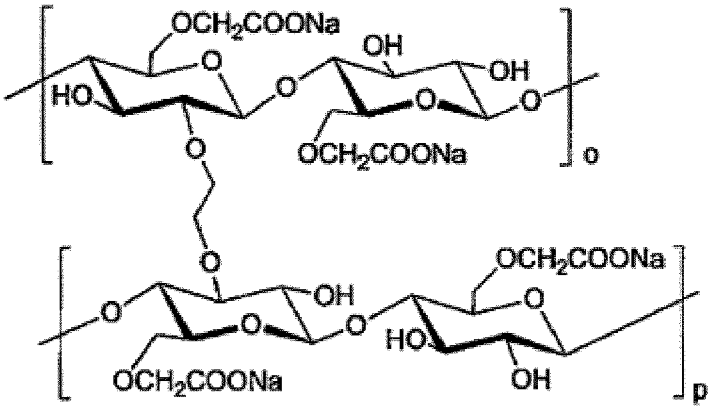

, und

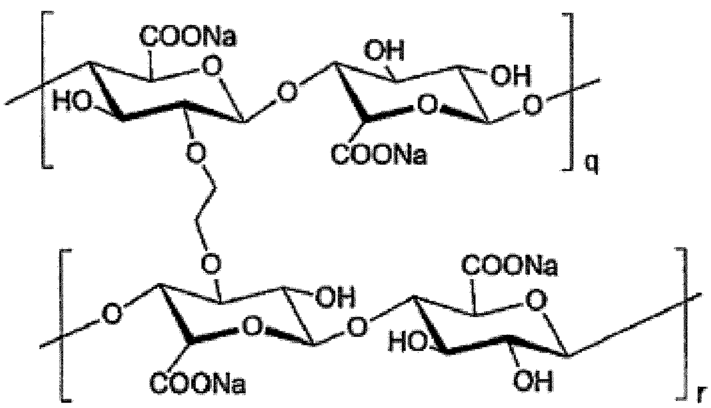

wobei m bis r 100 bis 200 sind.

8. Tumorversiegelungsmittel zur Verwendung nach einem der Ansprüche 1 bis 7, wobei 90 % oder mehr des Partikels eine Partikelgröße von 20 nm bis 500 nm aufweisen.

9. Tumorversiegelungsmittel zur Verwendung nach einem der Ansprüche 1 bis 8, wobei das Partikel in Form einer Emulsion vorliegt.

10. Tumorversiegelungsmittel zur Verwendung nach einem der Ansprüche 1 bis 9, wobei das Partikel ein Kontrastmittel und/oder einen Farbstoff umfasst.

11. Tumorversiegelungsmittel zur Verwendung nach einem der Ansprüche 1 bis 10, wobei das Tumorversiegelungsmittel in Form von gefriergetrockneten festen feinen Partikeln vorliegt.

**12.** Tumorversiegelungsmittel zur Verwendung nach einem der Ansprüche 1 bis 10, wobei das Tumorversiegelungsmittel in einer öligen Lösung vorliegt.

## Revendications

**1.** Agent de scellement pour tumeur comprenant une particule comprenant un composé polymère superabsorbant comme matériau de base, pour utilisation dans un procédé de traitement d'une tumeur maligne solide chez un sujet présentant la tumeur maligne solide,

dans lequel 70 % ou plus des particules totales ont une taille de particule de 20 nm à 500 nm, telle que mesurée par un procédé de diffusion dynamique de la lumière ;
dans lequel la particule peut gonfler au contact de l'eau dans le corps du sujet et le taux de gonflement de la particule est d'au moins 2 à 1 000 fois le poids ou le volume de son matériau de base séché ;
dans lequel, dans le procédé, l'agent de scellement pour tumeur est introduit, à l'aide d'une seringue ou d'un microcathéter, pour réaliser une embolie de la tumeur et/ou empêcher un écoulement du sang de l'artère à proximité de la tumeur ; et
dans lequel, dans le procédé, la particule pénètre à travers des espaces entre des cellules endothéliales de vaisseaux tumoraux pour s'accumuler dans du tissu tumoral hors des vaisseaux sanguins, puis gonfle au contact de l'eau dans le corps du sujet pour former une composition gélifiée, bloquant ainsi l'apport d'oxygène et de nutriments à la tumeur et la transmission de facteurs inducteurs de la tumeur pour conduire les cellules tumorales à la nécrose.

**2.** Agent de scellement pour tumeur pour utilisation selon la revendication 1, dans lequel le composé polymère superabsorbant présente une structure représentée par ce qui suit :

PS1-X-PS2 (1)

dans laquelle PS1 et PS2 sont des polysaccharides et X est un espaceur.

**3.** Agent de scellement pour tumeur pour utilisation selon la revendication 2, dans lequel PS1 et PS2 peuvent être identiques ou différents et sont indépendamment choisis dans le groupe constitué par le β-glucane, la chitine, le chitosane, et des dérivés de ceux-ci et des mélanges de ceux-ci.

**4.** Agent de scellement pour tumeur pour utilisation selon la revendication 3, dans lequel le β-glucane ou le dérivé de celui-ci est la cellulose ou la carboxyméthylcellulose.

**5.** Agent de scellement pour tumeur pour utilisation selon l'une quelconque des revendications 2 à 4, dans lequel X est un ou plusieurs anhydrides d'acide polycarboxylique.

**6.** Agent de scellement pour tumeur pour utilisation selon l'une quelconque des revendications 2 à 4, dans lequel X est dérivé d'un ou de plusieurs agents de réticulation choisis dans le groupe constitué par : le dianhydride d'acide 1,2,3,4-butanetétracarboxylique (BTCA), l'éther diglycidylique de polyéthylène glycol (PEGDE), le dianhydride d'acide biphényltétracarboxylique (BPDA), le dianhydride d'acide diphénylsulfonetétracarboxylique (DSDA), le PEG (polyéthyne glycol) 400, le PEG 2000 et l'épichlorhydrine.

**7.** Agent de scellement pour tumeur pour utilisation selon la revendication 1, dans lequel le composé polymère superabsorbant est un ou plusieurs choisis dans le groupe constitué par :

[Formule chimique 1]

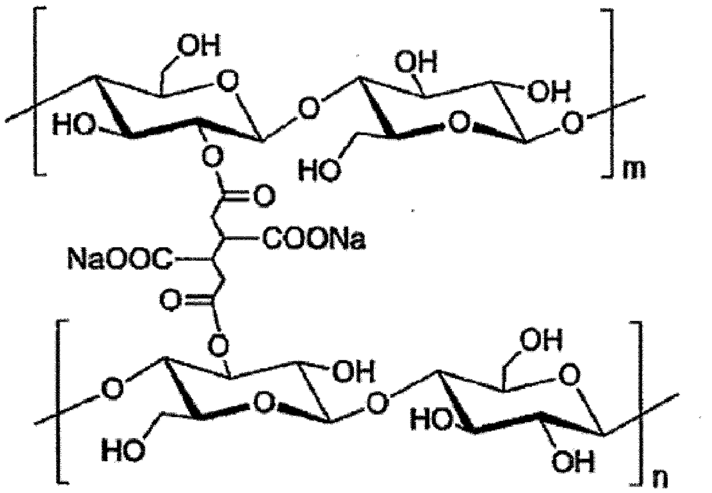

,

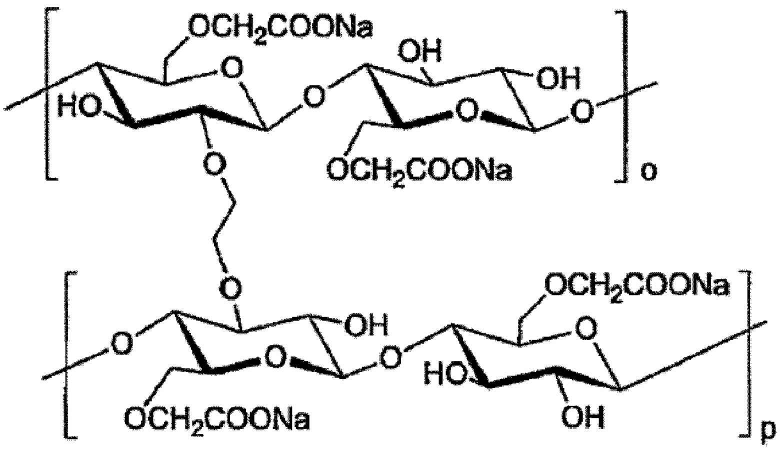

, et

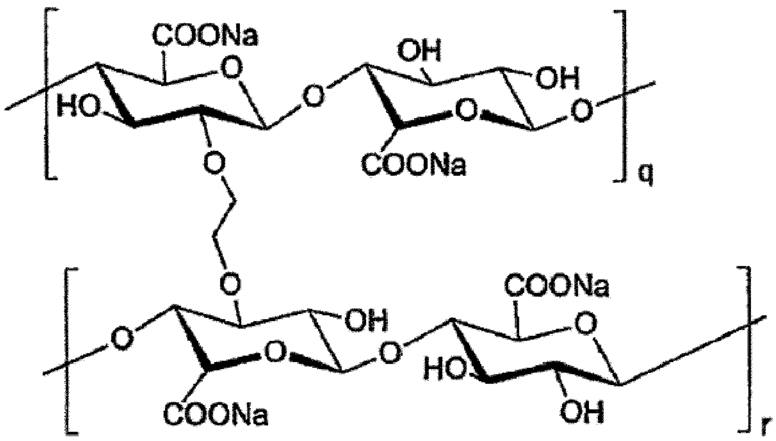

où m à r sont compris entre 100 et 200.

8. Agent de scellement pour tumeur pour utilisation selon l'une des revendications 1 à 7, dans lequel 90 % ou plus de la particule a une taille comprise entre 20 nm et 500 nm.

9. Agent de scellement pour tumeur pour utilisation selon l'une des revendications 1 à 8, dans lequel la particule est sous forme d'émulsion.

**10.** Agent de scellement pour tumeur pour utilisation selon l'une des revendications 1 à 9, dans lequel la particule comprend un agent de contraste et/ou un colorant.

**11.** Agent de scellement pour tumeur pour utilisation selon l'une des revendications 1 à 10, dans lequel l'agent de scellement pour tumeur est sous la forme de fines particules solides lyophilisées.

**12.** Agent de scellement pour tumeur pour utilisation selon l'une des revendications 1 à 10, dans lequel l'agent de scellement pour tumeur est dans une solution huileuse.

FIG. 1

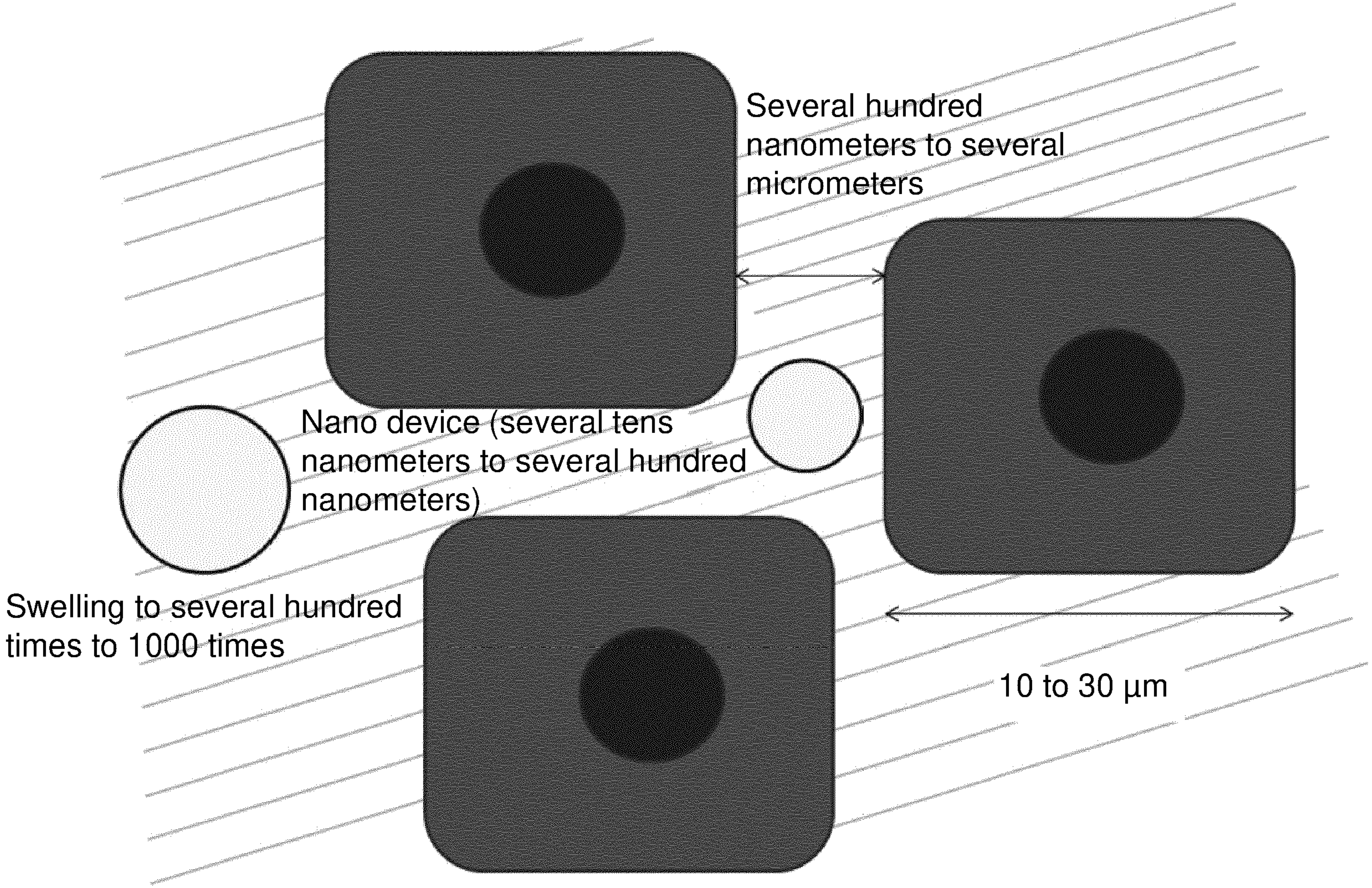
Several hundred nanometers to several micrometers
Nano device (several tens nanometers to several hundred nanometers)
Swelling to several hundred times to 1000 times
10 to 30 µm

FIG. 2

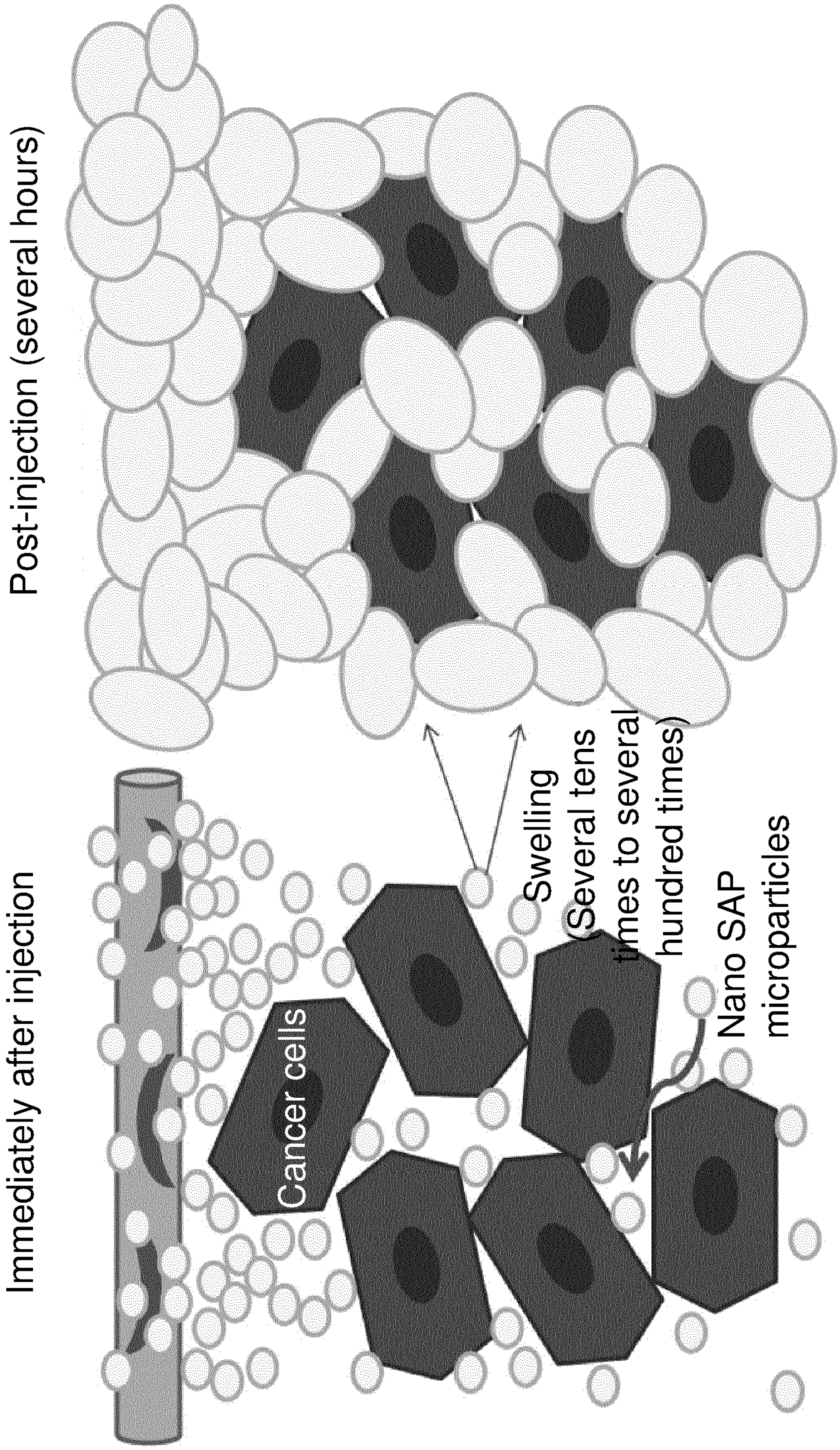
Immediately after injection
Post-injection (several hours)
Cancer cells
Swelling
(Several tens times to several hundred times)
Nano SAP microparticles

FIG. 3

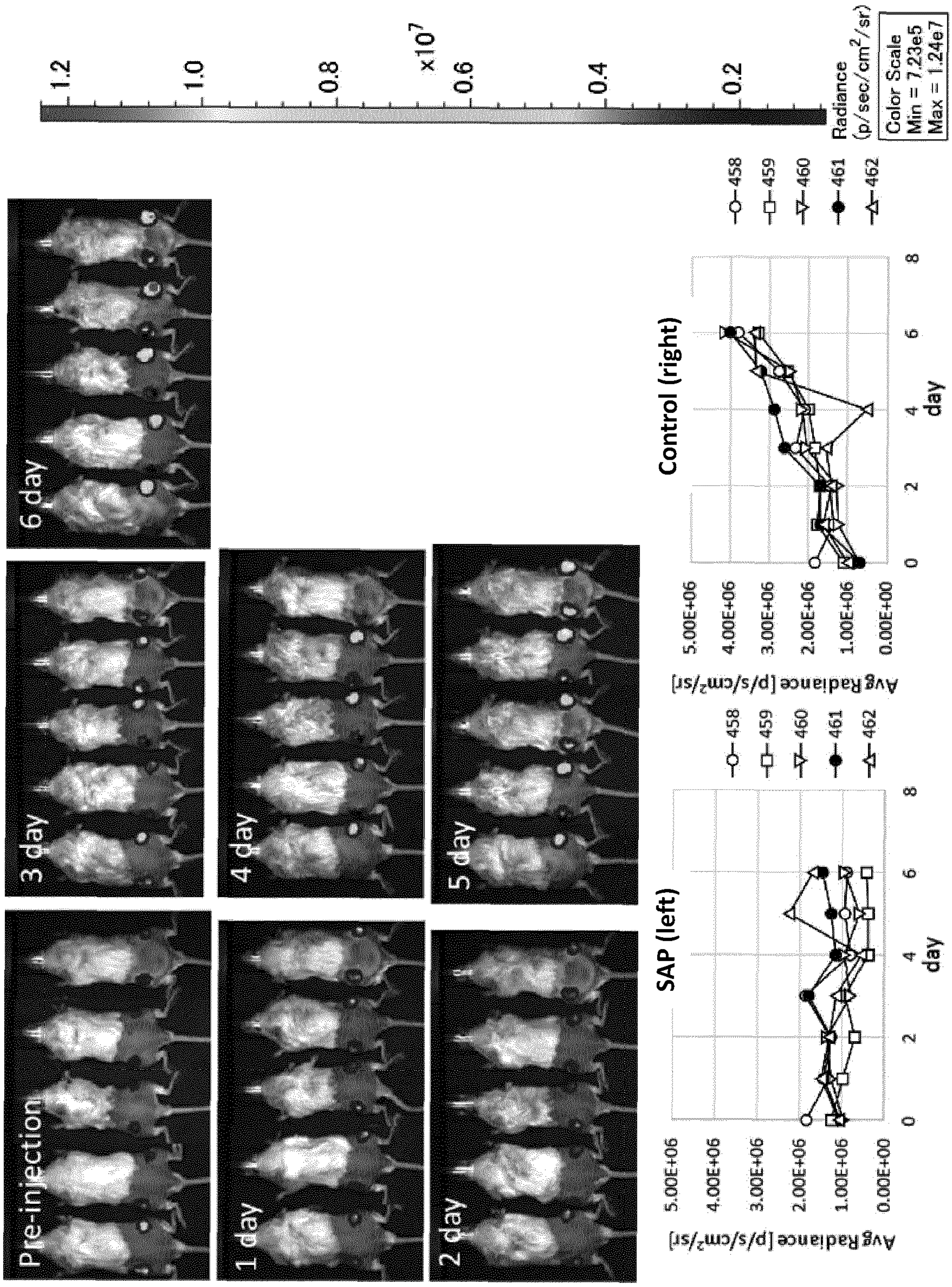
Pre-injection
1 day
2 day
3 day
4 day
5 day
6 day
SAP (left)
Control (right)
Avg Radiance [p/s/cm²/sr]
5.00E+06
4.00E+06
3.00E+06
2.00E+06
1.00E+06
0.00E+00
0
2
4
6
8
day
458
459
460
461
462
1.2
1.0
0.8
0.6
0.4
0.2
x10⁷
Radiance
(p/sec/cm²/sr)
Color Scale
Min = 7.23e5
Max = 1.24e7

FIG. 4

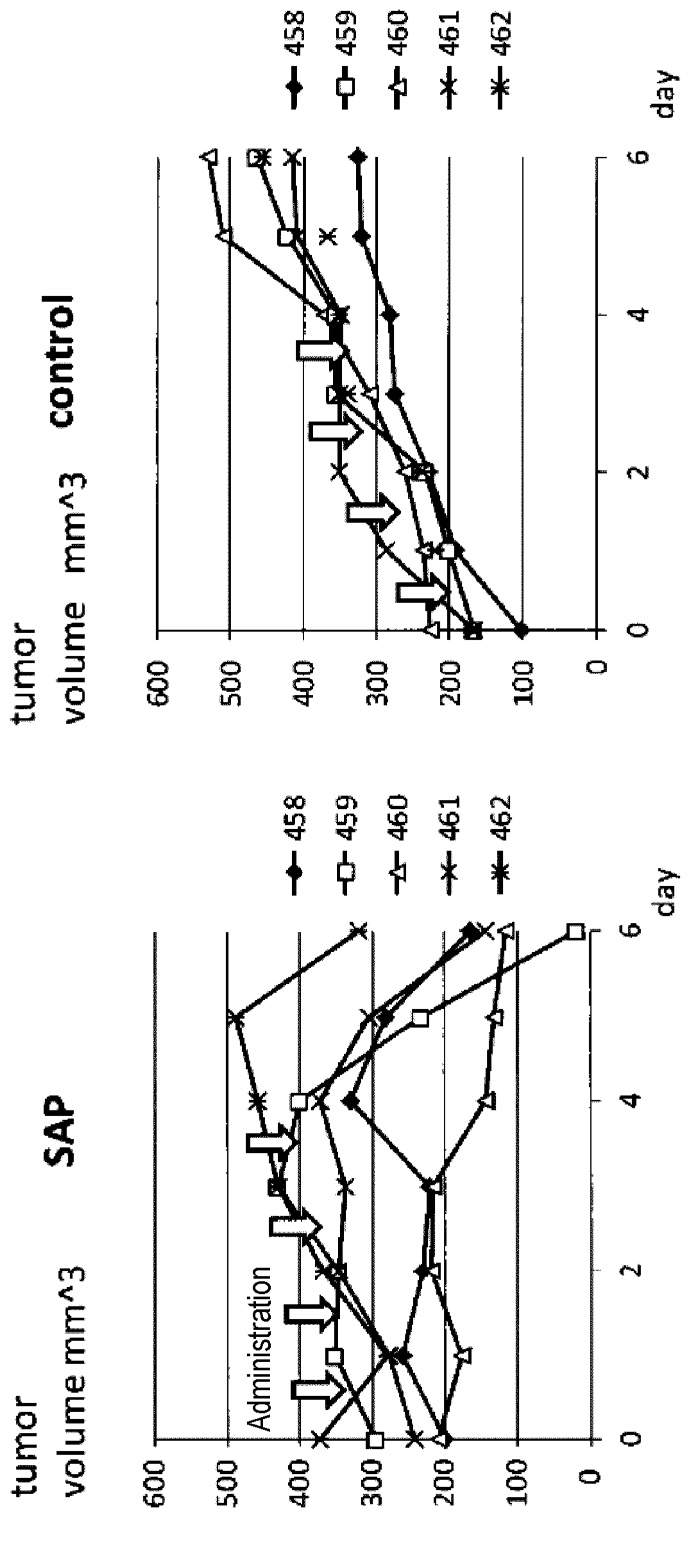
SAP
tumor volume mm^3
Administration
600
500
400
300
200
100
0
0
2
4
6
day
458
459
460
461
462
control
tumor volume mm^3
600
500
400
300
200
100
0
0
2
4
6
day
458
459
460
461
462

# FIG. 5

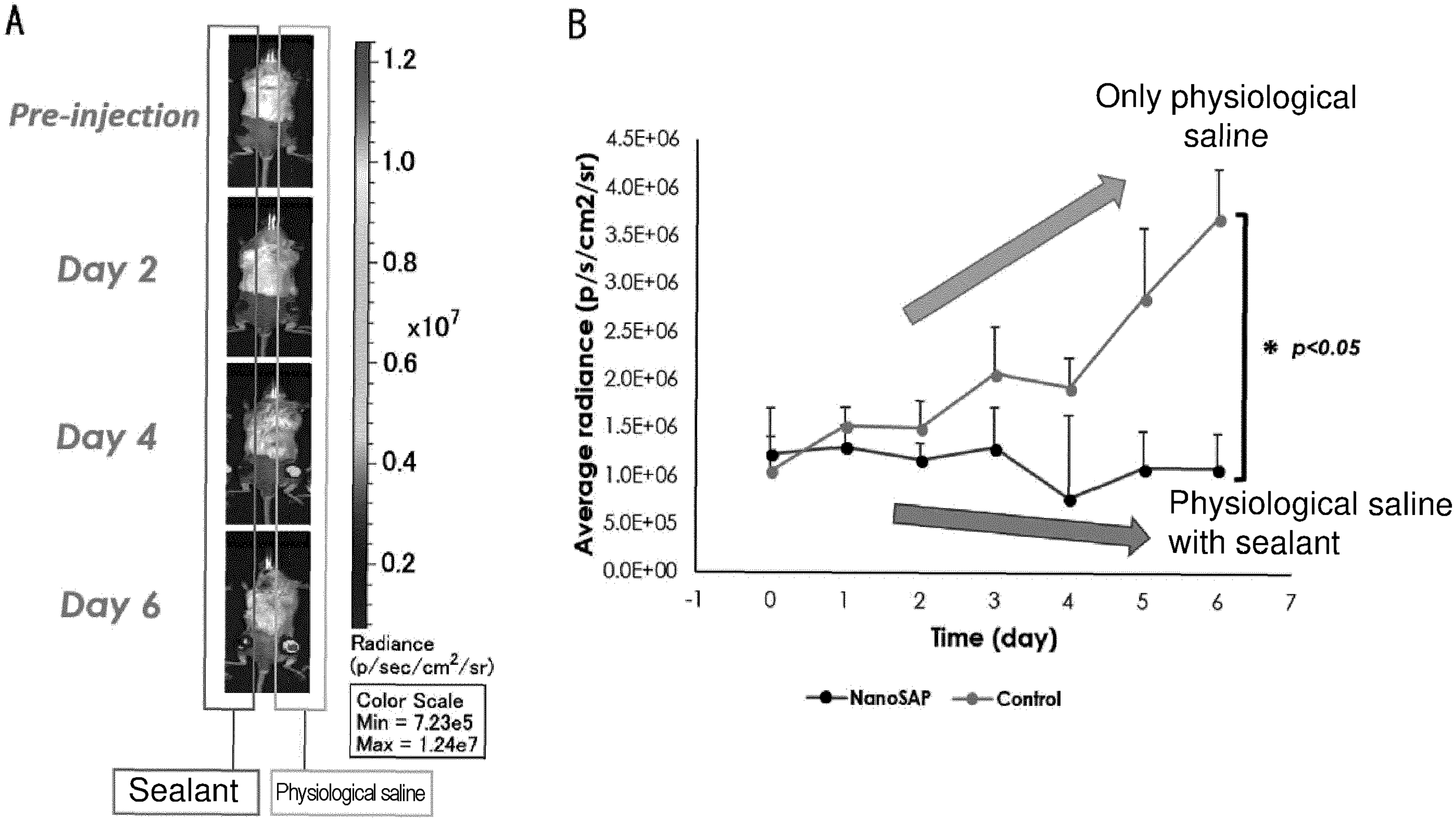
A
Pre-injection
Day 2
Day 4
Day 6
Sealant
Physiological saline
1.2
1.0
0.8
x10^7
0.6
0.4
0.2
Radiance (p/sec/cm^2/sr)
Color Scale
Min = 7.23e5
Max = 1.24e7
B
Only physiological saline
Physiological saline with sealant
* p<0.05
Average radiance (p/s/cm2/sr)
4.5E+06
4.0E+06
3.5E+06
3.0E+06
2.5E+06
2.0E+06
1.5E+06
1.0E+06
5.0E+05
0.0E+00
-1
0
1
2
3
4
5
6
7
Time (day)
NanoSAP
Control

# FIG. 6

## A. Example with physiological saline vehicle

| | Immediately before administration % ($mm^3$) | Day 8 post-administration % ($mm^3$) | Extracted tumor |
|---|---|---|---|
| Control side (left) | 100% (265.2) | 342% (907.5) | 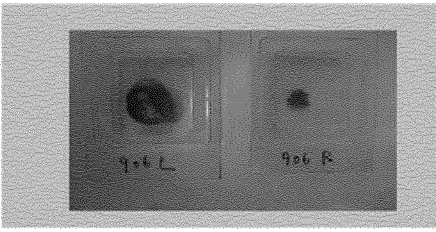 |
| MD3 administration side (right) | 100% (103.5) | 69% (71.5) | |
| Ratio of tumor volume of MD3 administration side to volume of control side (%) | 39.0 | 7.9 | |

## B. Example with physiological saline + Lipiodol vehicle

| | Immediately before administration % ($mm^3$) | Day 8 post-administration % ($mm^3$) | Extracted tumor |
|---|---|---|---|
| Control side (left) | 100% (202.3) | 126% (255.1) | 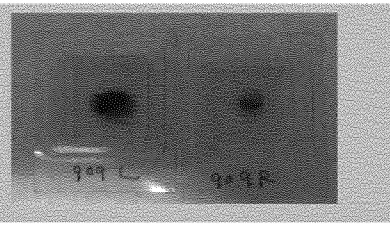 |
| MD3 administration side (right) | 100% (189.6) | 14% (25.7) | |
| Ratio of tumor volume of MD3 administration side to volume of control side (%) | 93.7% | 10.1% | |

FIG. 7

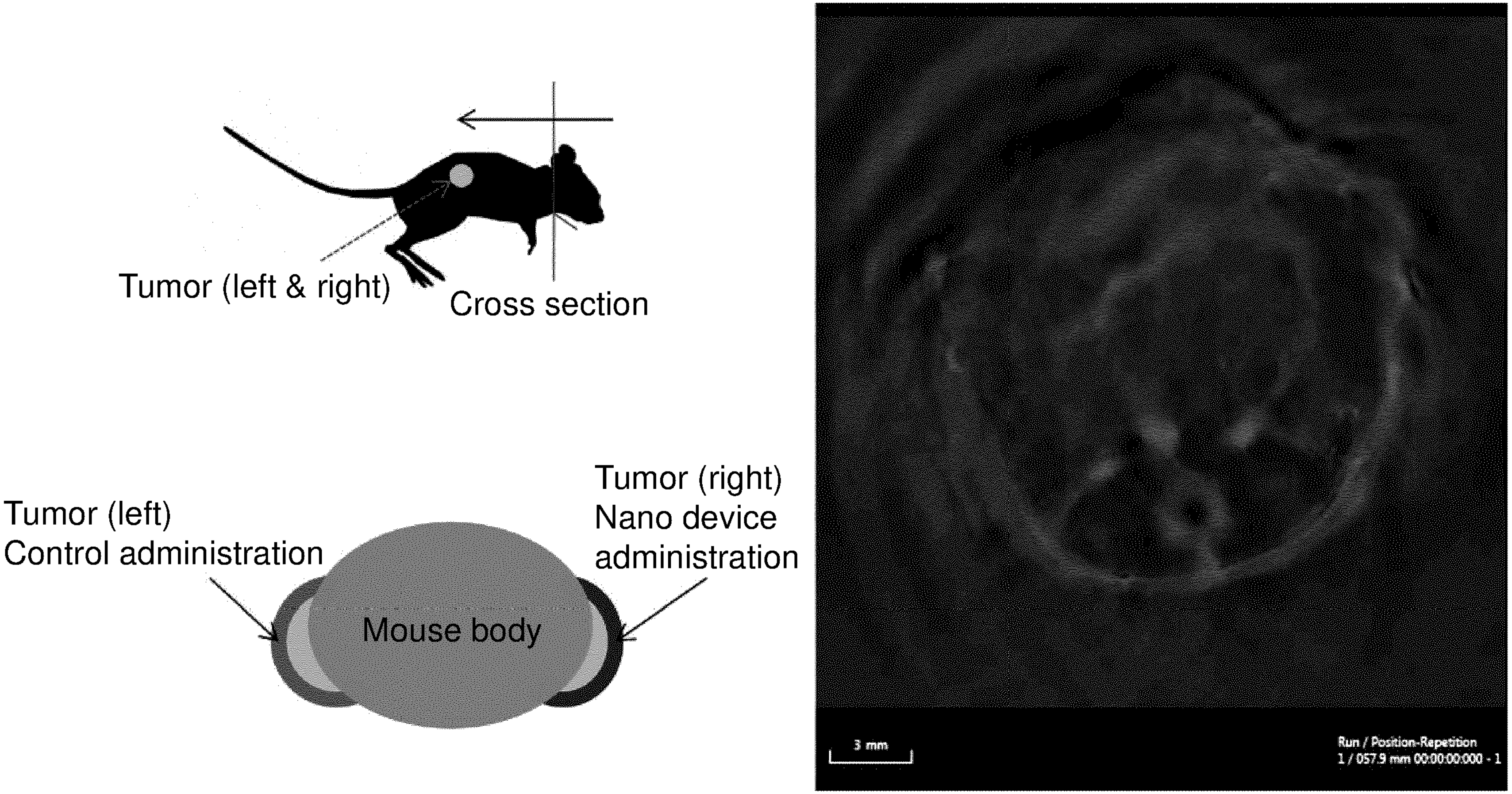
Tumor (left & right)
Cross section
Tumor (left)
Control administration
Mouse body
Tumor (right)
Nano device
administration
3 mm
Run / Position-Repetition
1 / 057.9 mm 00:00:00:000 - 1

FIG. 8

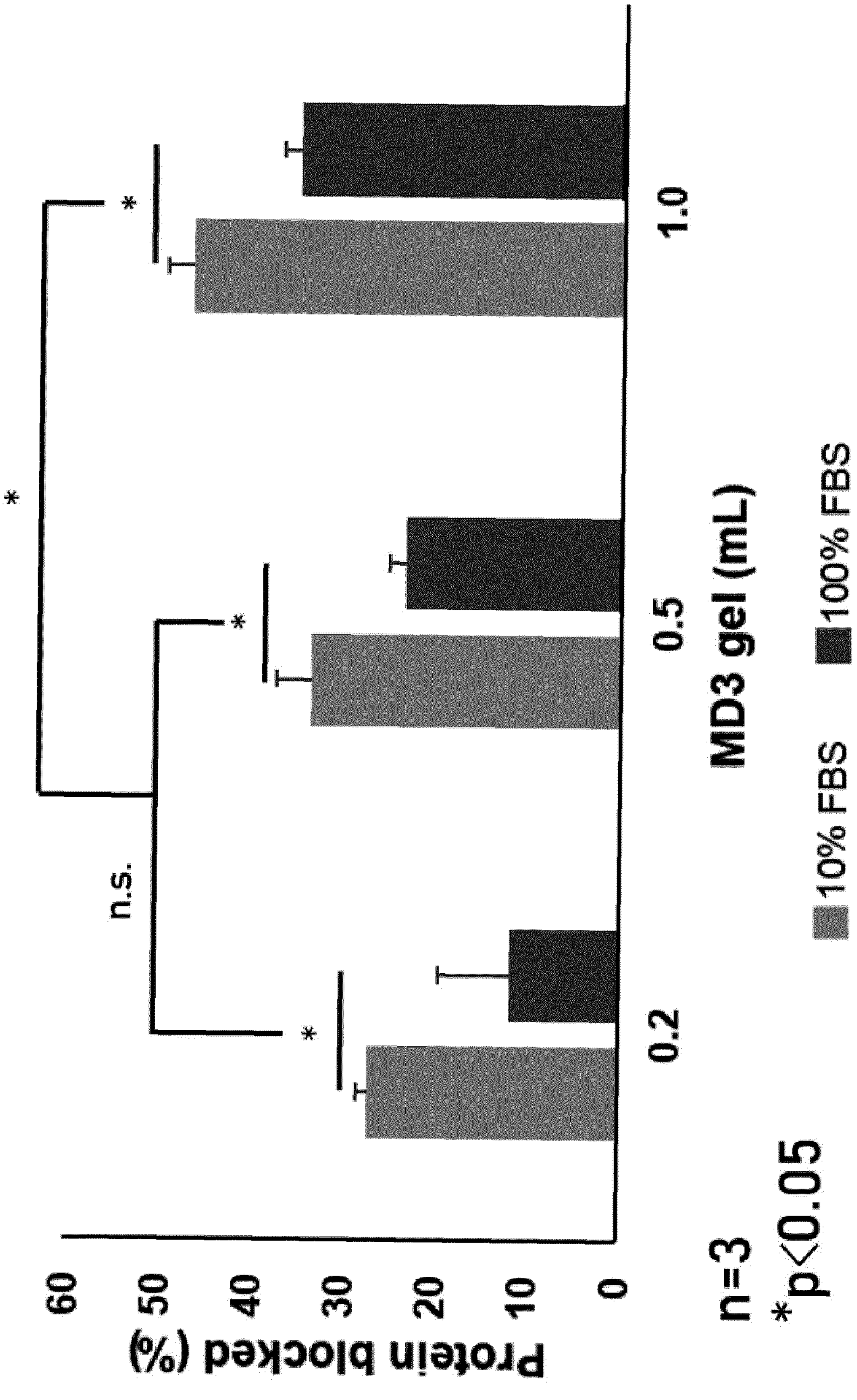
Protein blocked (%)
60
50
40
30
20
10
0
n.s.
*
*
*
*
0.2
0.5
1.0
MD3 gel (mL)
10% FBS
100% FBS
n=3
*p<0.05

FIG. 9

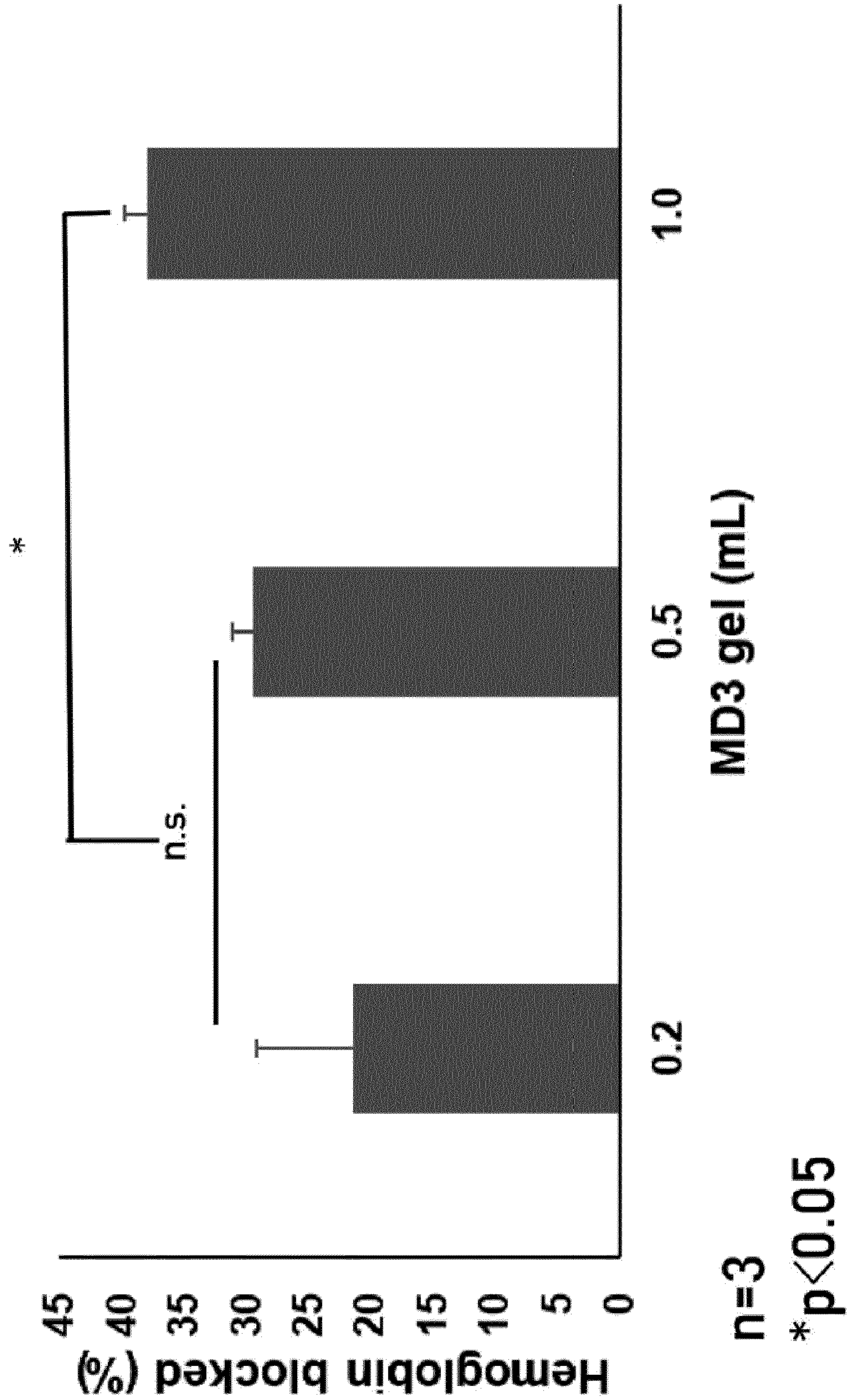
Hemoglobin blocked (%)
45
40
35
30
25
20
15
10
5
0
n.s.
*
0.2
0.5
1.0
MD3 gel (mL)
n=3
*p<0.05

FIG. 10

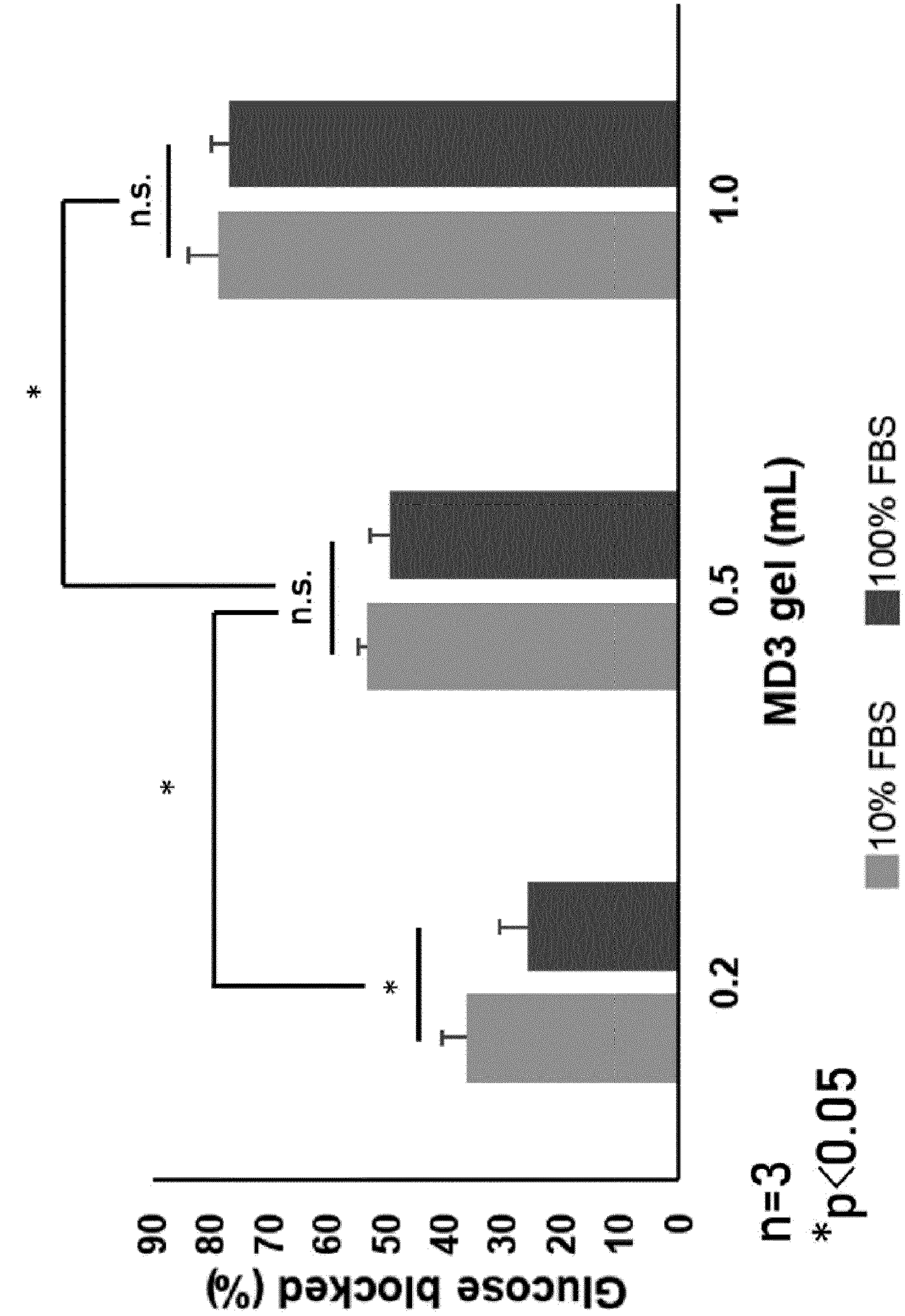
Glucose blocked (%)
90
80
70
60
50
40
30
20
10
0
0.2
0.5
1.0
MD3 gel (mL)
n.s.
n.s.
*
*
*
10% FBS
100% FBS
n=3
*p<0.05

FIG. 11

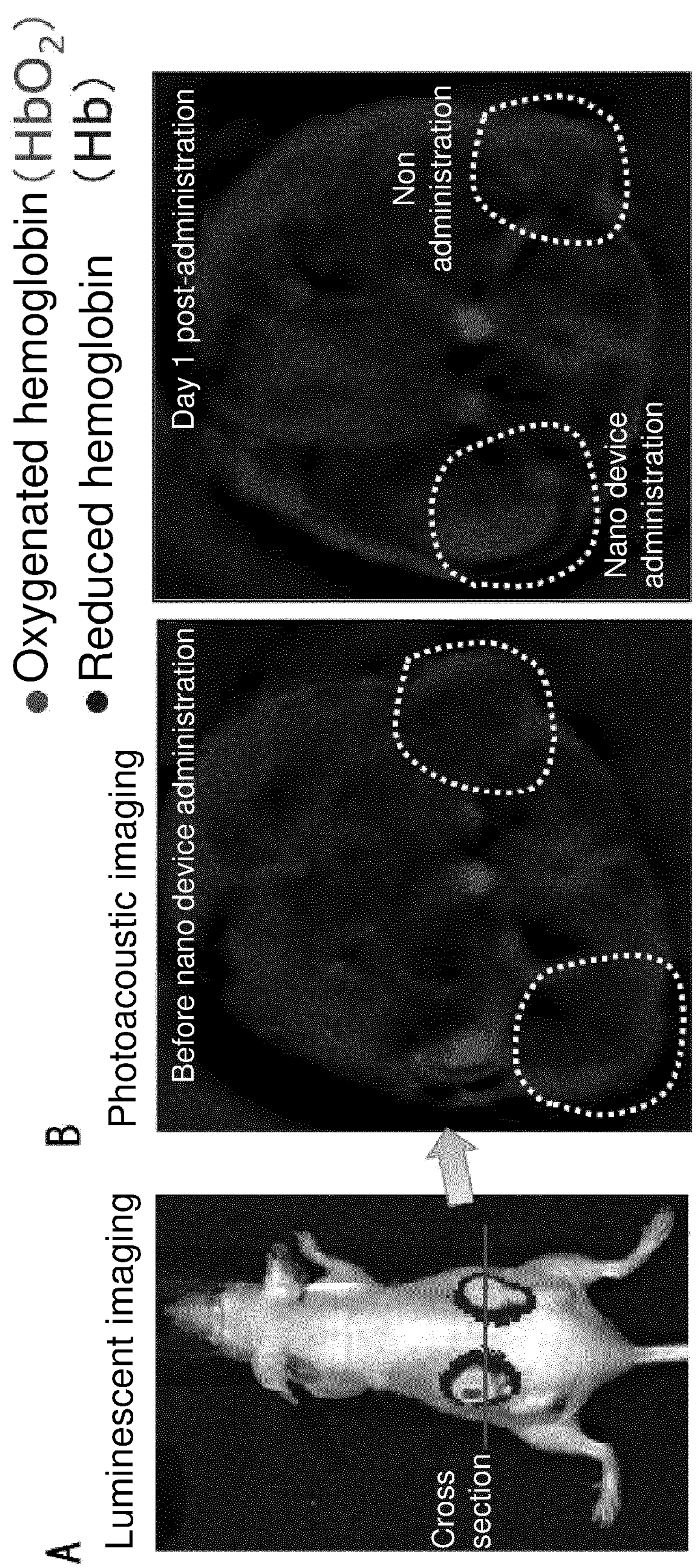
A
Luminescent imaging
Cross section
4T1 cell-transplant mouse
B
Photoacoustic imaging
Before nano device administration
Day 1 post-administration
Nano device administration
Non administration
Oxygenated hemoglobin ($HbO_2$)
Reduced hemoglobin (Hb)

FIG. 12

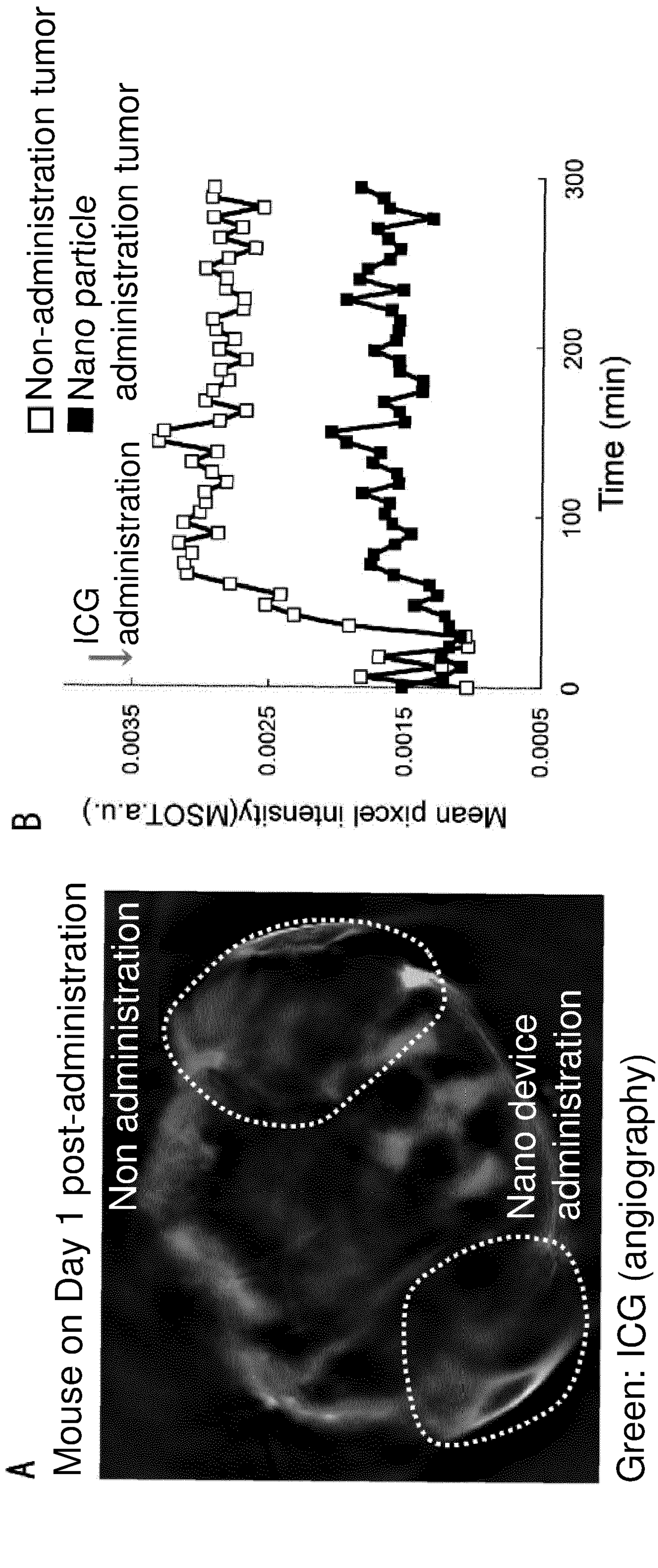
A
Mouse on Day 1 post-administration
Non administration
Nano device administration
Green: ICG (angiography)
B
Mean pixcel intensity(MSOT a.u.)
0.0035
0.0025
0.0015
0.0005
ICG administration
Non-administration tumor
Nano particle administration tumor
0
100
200
300
Time (min)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004313759 A
- WO 2011078383 A
- WO 2014178256 A
- CN 103040741
- CN 101822635
- CN 103536974
- WO 2017104840 A
- US 20070237830 A1
- WO 2012147255 A
- JP 2012012462 A


### Non-patent literature cited in the description


- An Indirect Way to Tame Cancer. **R. K. JAIN.** Scientific American. November 2014, 61
- **YUN BIN WU.** Research and development of new drugs. *Development of study on ''cubic liquid crystal in controlled release preparation,* 06 September 2010, (48